# EUROPEAN PATENT APPLICATION

(11) **EP 4 470 629 A1**
(43) Date of publication of application: **04.12.2024**
(21) Application number: 23746826.9
(22) Date of filing: 19.01.2023
(51) Int. Cl.: A61Q 17/04, A61K 8/06, A61K 8/25, A61K 8/31, A61K 8/34, A61K 8/362, A61K 8/37, A61K 8/40, A61K 8/49, A61K 8/73, A61K 8/898

(54) **OIL-IN-WATER EMULSION SKIN PREPARATION FOR EXTERNAL USE**

(30) Priority: 28.01.2022 US 202263304103 P
(71) Applicant: Kao Corporation, Tokyo 103-8210 (JP)
(72) Inventor: FUJII Tomoya, Tokyo 131-8501 (JP); HIRONO Shingo, Tokyo 131-8501 (JP)
(74) Representative: Hoffmann Eitle
(86) International application number: PCT/JP2023/001583
(87) International publication number: WO 2023/145613

(57) **Abstract**

Provided are an external preparation for skin of oil-in-water type emulsification containing components (A), (B), (C), and (D) below, and a method for producing the external preparation for skin of oil-in-water type emulsification.
(A): silica particles having an average particle diameter of 0.3 µm or more and 3 µm or less;
(B): at least one selected from the group consisting of (B1) a cationic polymer having a hydrophobic moiety, and (B2) a cationic surfactant having an alkyl group;
(C): an oily component; and
(D): an aqueous component.

## Description

### Field of the Invention

The present invention relates to an external preparation for skin of oil-in-water type emulsification.

### Background of the Invention

As a method for preparing an oil-in-water emulsion, an emulsification method using a surfactant and an emulsification method using a macromolecular emulsifier have been conventionally used. However, the method using a surfactant may reduce resistance to sweat and water, and the method using a macromolecular emulsifier may not sufficiently exert an emulsification ability depending upon the composition or amount of an inner oil phase.

Therefore, an emulsification method using fine particles as an emulsifier has been investigated.

For example, JP 2008-291026 A (Patent Literature 1) aims to provide an oil-in-water emulsion composition having excellent emulsion stability, and the like, and describes the oil-in-water emulsion composition that contains (a) 1 to 20 mass% of a powder component, (b) 0.001 to 0.5 mass% of a cationic surfactant having two alkyl chains each having 12 or more and 22 or less carbon atoms, (c) an oil phase component, and (d) a water phase component, has a structure in which the powder particles of (a) are adsorbed on oil droplets dispersed in the water phase, and does not substantially contain a surfactant other than (b) the cationic surfactant.

JP 2005-527548 A (Patent Literature 2) relates to a particle stabilizing composition suitable for skin care products and the like, and describes the particle stabilizing composition that contains a. an emulsion having an internal phase in an amount of about 1% by weight to about 99% by weight of the emulsion, and an external phase in an amount of about 1% by weight to about 99% by weight of the emulsion, b. a charged species present in the emulsion, and c. charged insoluble solid particles dispersed in the emulsion, in which the charged species possesses a charge opposed to that of the charged insoluble solid particles, essentially all of the charged species and the charged insoluble solid particles accumulate at the interface of the emulsion, and Brownian motion is not exhibited by the charged insoluble solid particles.

### Summary of the Invention

The present invention relates to an external preparation for skin of oil-in-water type emulsification containing components (A), (B), (C), and (D) below:
(A): silica particles having an average particle diameter of 0.3 µm or more and 3 µm or less;
(B): at least one selected from the group consisting of (B1) a cationic polymer having a hydrophobic moiety, and (B2) a cationic surfactant having an alkyl group;
(C): an oily component; and
(D): an aqueous component.

### Brief Description of the Drawing

FIG. 1 is a photomicrograph illustrating the emulsion state of an external preparation for skin of oil-in-water type emulsification obtained in Example 1.
FIG. 2 is a photomicrograph illustrating the emulsion state of a dilution in which the external preparation for skin of oil-in-water type emulsification obtained in Example 1 is diluted 50 times with water.
FIG. 3 is a photomicrograph illustrating the emulsion state before stirring in evaluation of emulsion stability in Example 1.
FIG. 4 is a photomicrograph illustrating the emulsion state after stirring in evaluation of emulsion stability in Example 1.
FIG. 5 is a photomicrograph illustrating the emulsion state before stirring in evaluation of emulsion stability in Comparative Example 1.
FIG. 6 is a photomicrograph illustrating the emulsion state after stirring in evaluation of emulsion stability in Comparative Example 1.

### Detailed Description of the Invention

Patent Literature 1 describes that the powder component preferably has a particle diameter of 1 to 200 nm, and in Examples, silica-coated titanium oxide having a particle diameter of 10 nm, silica-coated zinc oxide having a particle diameter of 30 nm, and silica having a particle diameter of 10 nm are used. In a technique of Patent Literature 2, titanium oxide is used as the charged insoluble solid particles.

However, it is shown that by the techniques of Patent Literature 1 and Patent Literature 2, an emulsion state immediately after preparation may be heterogeneous and emulsion stability may be insufficient. Therefore, further improvements in an emulsion state immediately after preparation and emulsion stability are required.

The present invention relates to an external preparation for skin of oil-in-water type emulsification that is excellent in an emulsion state immediately after preparation and emulsion stability.

The inventors of the present invention have focused on a stable oil-in-water type emulsion system that can be formed by incorporation of silica particles having an average particle diameter of a predetermined range, and at least one selected from the group consisting of a cationic polymer having a hydrophobic moiety and a cationic surfactant having an alkyl group, and found that an external preparation for skin of oil-in-water type emulsification that is excellent in an emulsion state immediately after preparation and emulsion stability can be provided.

Specifically, the present invention relates to the following [1] and [2].
[1] An external preparation for skin of oil-in-water type emulsification, containing components (A), (B), (C), and (D) below:
   (A): silica particles having an average particle diameter of 0.3 µm or more and 3 µm or less;
   (B): at least one selected from the group consisting of (B 1) a cationic polymer having a hydrophobic moiety, and (B2) a cationic surfactant having an alkyl group;
   (C): an oily component; and
   (D): an aqueous component.
[2] A method for producing the external preparation for skin of oil-in-water type emulsification according to [1], the method including a step 1 of mixing the component (A) with the component (B) in water.

The present invention can provide an external preparation for skin of oil-in-water type emulsification that is excellent in an emulsion state immediately after preparation and emulsion stability.

### [External Preparation for Skin of Oil-In-Water Type Emulsification]

An external preparation for skin of oil-in-water type emulsification of the present invention (hereinafter also referred to as "external preparation for skin of the present invention", "external preparation for skin", or "external preparation for skin of oil-in-water type emulsification") contains the following components (A), (B), (C), and (D):
(A): silica particles having an average particle diameter of 0.3 µm or more and 3 µm or less;
(B): at least one selected from the group consisting of (B1) a cationic polymer having a hydrophobic moiety, and (B2) a cationic surfactant having an alkyl group;
(C): an oily component; and
(D): an aqueous component.

The "average particle diameter" in the present invention is a volume average particle diameter (D₅₀) measured with a laser diffraction/scattering particle diameter dispersion measurement device "LA-960" (manufactured by Horiba, Ltd.) after 0.1 g of the component (A) is added to 10 g of purified water and the mixture is subjected to ultrasonic dispersion for 1 minute.

The external preparation for skin of oil-in-water type emulsification of the present invention is excellent in an emulsion state immediately after preparation and emulsion stability. Though not clear, the reason is considered to be as follows.

The external preparation for skin of oil-in-water type emulsification of the present invention contains silica particles having an average particle diameter of 0.3 µm or more and 3 µm or less and at least one type of cationic compound selected from the group consisting of a cationic polymer having a hydrophobic moiety and a cationic surfactant having an alkyl group. In water, particles in which the cationic compound is adsorbed to the silica particles are formed. The particles are amphiphilic silica particles having a hydrophilic property derived from a silanol group on the surface of the silica particles and a hydrophobic property derived from the hydrophobic moiety or alkyl group of the cationic compound. It is considered that under alignment of the amphiphilic silica particles at the interface between a water phase and an oil phase, a stable oil-in-water type emulsion system can be formed, and as a result, an emulsion state immediately after preparation and emulsion stability are improved.

Herein, in the present invention, "adsorption" in the particles in which the cationic compound is adsorbed to the silica particles is not particularly limited, and may be chemisorption such as electrostatic attraction, or physical adsorption such as hydrophobic adsorption by Van der Waals force.

### <Component (A)>

From the viewpoint of rapidly forming a stable oil-in-water type emulsion system to improve an emulsion state immediately after preparation and emulsion stability, the external preparation for skin of the present invention contains as the component (A) silica particles having an average particle diameter of 0.3 µm or more and 3 µm or less.

In the present invention, the silica particles as the component (A) are preferably aligned at the interface between the water phase and the oil phase. In other words, the external preparation for skin of the present invention is preferably in the form of so-called Pickering emulsion in which in the oil-in-water type emulsion system, the silica particles as the component (A) are aligned at the interface between the water phase and the oil phase and the oil phase is emulsified in the water phase. The alignment of the silica particles as the component (A) in the external preparation for skin of the present invention at the interface between the water phase and the oil phase can be confirmed by observation with a microscope.

The component (A) is particles containing as a main component silica (silicon dioxide).

The component (A) is a synthetic or natural product, and is crystalline or amorphous. In particular, the component (A) is preferably synthetic silica particles. Examples of a method for producing synthetic silica particles include wet methods such as a precipitation method and a gelation method; and dry methods such as a combustion method and an arc method.

The component (A) may be hydrophilic silica particles or hydrophobic silica particles.

In the description, the "hydrophilic silica particles" mean silica particles having a silanol (-SiOH) group on the surface thereof (i.e., surface-untreated silica particles in which the surface is not hydrophobized). The "hydrophobic silica particles" mean surface-hydrophobized silica particles in which the surface is hydrophobized with an alkylsilane or the like.

When as the component (A), hydrophilic silica particles are used, the surface of the component (A) is negatively charged in water. Therefore, chemisorption by electrostatic attraction between the negative charge of the component (A) and the positive charge of the component (B) is predominant, and particles in which the component (B) is adsorbed to the component (A) are formed. It is considered that the particles are amphiphilic silica particles having a hydrophilic property derived from a silanol group on the surface of the component (A) and a hydrophobic property derived from the hydrophobic moiety or the alkyl group of the component (B), and under alignment of the amphiphilic silica particles at the interface between a water phase and an oil phase, a stable oil-in-water type emulsion system can be formed, and as a result, an emulsion state immediately after preparation and emulsion stability are improved.

When as the component (A), the hydrophobic silica particles are used, particles in which the component (B) is adsorbed to the component (A) are formed by physical adsorption such as hydrophobic adsorption by the Van der Waals force between a hydrophobic functional group such as the alkyl group on the surface of the component (A) and the hydrophobic moiety or the alkyl group of the component (B). It is considered that the particles are amphiphilic silica particles having a hydrophilic property derived from a silanol group remaining on the surface of the component (A) and a cationic moiety of the component (B) and a hydrophobic property derived from a hydrophobized moiety of the component (A) and the hydrophobic moiety of the component (B), and under alignment of the amphiphilic silica particles at the interface between a water phase and an oil phase, a stable oil-in-water type emulsion system can be formed, and as a result, an emulsion state immediately after preparation and emulsion stability are improved.

In particular, the component (A) is preferably hydrophilic silica particles (that is, surface-untreated silica particles) from the viewpoint of rapidly forming a stable oil-in-water type emulsion system to improve an emulsion state immediately after preparation and emulsion stability.

When the component (A) is hydrophilic silica particles, the hydrophilic silica particles may contain an inorganic element other than Si, such as Al, and the content of silica (SiO₂) in the hydrophilic silica particles is preferably 90 mass% or more, more preferably 95 mass% or more, and even more preferably 99 mass% or more, and preferably 100 mass% or less.

The definition of the average particle diameter of the component (A) is as described above.

From the viewpoint of rapidly forming a stable oil-in-water type emulsion system to improve an emulsion state immediately after preparation and emulsion stability, the average particle diameter of the component (A) is preferably 0.4 µm or more, more preferably 0.5 µm or more, and even more preferably 0.6 µm or more, and preferably 2.9 µm or less, and more preferably 2.8 µm or less. More specifically, from the viewpoint of rapidly forming a stable oil-in-water type emulsion system to improve an emulsion state immediately after preparation and emulsion stability, the average particle diameter of the component (A) is preferably 0.4 µm or more and 3 µm or less, more preferably 0.5 µm or more and 3 µm or less, even more preferably 0.5 µm or more and 2.9 µm or less, and still even more preferably 0.6 µm or more and 2.8 µm or less.

The average particle diameter of the component (A) is measured according to a method described in Examples.

The amount of oil absorption of the component (A) is preferably 5 mL/100 g or more, more preferably 10 mL/100 g or more, and even more preferably 20 mL/100 g or more, and preferably 700 mL/100 g or less, more preferably 600 mL/100 g or less, even more preferably 500 mL/100 g or less, even more preferably 300 mL/100 g or less, even more preferably 200 mL/100 g or less, even more preferably 170 mL/100 g or less, and still even more preferably 150 mL/100 g or less. The amount of oil absorption can be measured in accordance with JIS K 5101-13-2:2004.

Examples of the particle structure of the component (A) include a porous particle and a non-porous particle. In the present invention, the "porous particle" is a particle having high specific surface area, and means a particle having a specific surface area of more than 50 m²/g, and the "non-porous particle" is a particle having low specific surface area, and means a particle having a specific surface area of 50 m²/g or less. The specific surface area can be measured by a BET method in accordance with JIS Z 8830:2013.

Examples of commercially available products of the component (A) include "SILICA MICRO BEAD" series (available from JGC Catalysts and Chemicals Ltd.) and "Godd Ball" series (available from SUZUKIYUSHI INDUSTRIAL CORPORATION).

From the viewpoint of rapidly forming a stable oil-in-water type emulsion system to improve an emulsion state immediately after preparation and emulsion stability, the content of the component (A) in the external preparation for skin of the present invention is preferably 0.3 mass% or more, more preferably 0.5 mass% or more, even more preferably 1 mass% or more, and even more preferably 2 mass% or more, and preferably 13 mass% or less, more preferably 10 mass% or less, even more preferably 7 mass% or less, even more preferably 5 mass% or less, and still even more preferably 4 mass% or less. More specifically, from the viewpoint of rapidly forming a stable oil-in-water type emulsion system to improve an emulsion state immediately after preparation and emulsion stability, the content of the component (A) in the external preparation for skin of the present invention is preferably 0.3 to 13 mass%, more preferably 0.5 to 10 mass%, even more preferably 1 to 10 mass%, even more preferably 1 to 7 mass%, even more preferably 1 to 5 mass%, even more preferably 2 to 5 mass%, and still even more preferably 2 to 4 mass%.

### <Component (B)>

From the viewpoint of rapidly forming a stable oil-in-water type emulsion system to improve an emulsion state immediately after preparation and emulsion stability, the external preparation for skin of emulsification of the present invention contains as the component (B) at least one selected from the group consisting of (B 1) a cationic polymer having a hydrophobic moiety (hereinafter also referred to as "component (B1)"), and (B2) a cationic surfactant having an alkyl group (hereinafter also referred to as "component (B2)").

In the present invention, the "cationic group" refers to a cationic group or a group that can be ionized to become a cationic group. Examples of the cationic group of the component (B) include a primary amino group, a secondary amino group, a tertiary amino group, and a quaternary ammonium group.

In the present invention, from the viewpoint of rapidly forming a stable oil-in-water type emulsion system to improve an emulsion state immediately after preparation and emulsion stability, the form of existence of the component (A) and the component (B) in the external preparation for skin of oil-in-water type emulsification is preferably the form of particles in which the cationic polymer or cationic surfactant as the component (B) is adsorbed to the silica particles of the component (A).

Here, "adsorption" in the particles in which the component (B) is adsorbed to the component (A) is not particularly limited as described above, and may be chemisorption such as electrostatic attraction, or physical adsorption such as hydrophobic adsorption by Van der Waals force. It is considered that when the component (A) is hydrophilic silica particles, chemisorption by electrostatic attraction is predominant, and when the component (A) is hydrophobic silica particles, physical adsorption such as hydrophobic adsorption by Van der Waals force is predominant.

### [(B1) Cationic Polymer Having Hydrophobic Moiety]

In the present invention, the "cationic polymer" is a polymer that has a cationic group and exhibits cationic properties as a whole.

From the viewpoint of improving an emulsion state immediately after preparation and emulsion stability, the cationic group of the component (B1) is preferably a quaternary ammonium group.

Preferred examples of the hydrophobic moiety of the component (B1) include a polysiloxane moiety; a hydrocarbon moiety such as an alkyl group, an alkenyl group, an aryl group, and an aralkyl group; and an alkyleneoxy moiety having 3 or more carbon atoms. The component (B 1) may have one or two or more types of hydrophobic moieties. Among these, from the viewpoint of improving an emulsion state immediately after preparation and emulsion stability, the hydrophobic moiety of the component (B1) is preferably at least one type of hydrophobic moiety selected from the group consisting of a polysiloxane moiety, a hydrocarbon moiety, and an alkyleneoxy moiety having 3 or more carbon atoms.

From the viewpoint of improving an emulsion state immediately after preparation and emulsion stability, the component (B 1) is preferably at least one selected from the group consisting of a cationic silicone, a cationic vinyl polymer, and a cationized cellulose derivative.

As the component (B 1), one may be used alone or two or more may be used in combination.

### (Cationic Silicone)

A cationic silicone is preferably a silicone having at least one quaternary ammonium group at an end or side chain of organopolysiloxane. A specific example of the silicone having a quaternary ammonium group is preferably a quaternary ammonium cation-modified silicone having at least one structure selected from the group consisting of a structure represented by the following general formula (1-1) and a structure represented by the following general formula (1-2) (hereinafter also simply referred to as "quaternary ammonium cation-modified silicone").

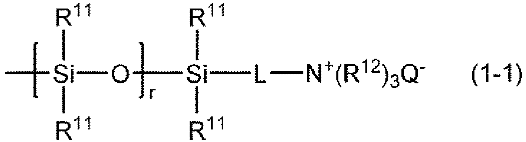

In the formula (1-1), each R¹¹ represents a hydrocarbon group having 1 or more and 6 or less carbon atoms, each R¹² represents a hydrogen atom, a hydrocarbon group having 1 or more and 20 or less carbon atoms, or an amide bond-containing hydrocarbon group having 1 or more and 20 or less carbon atoms, L represents a divalent organic group, Q⁻ is a counterion of a quaternary ammonium ion, and r represents a number of 2 or more. A plurality of R¹¹'s and a plurality of R¹²'s may be the same or different. The bonding order of the parenthesized structural units is not limited, and the bonding form thereof may be a block form or a random form.

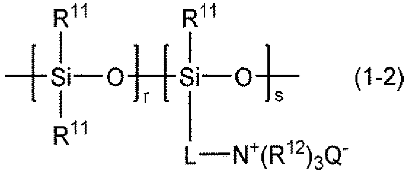

In the formula (1-2), R¹¹'s, R¹²'s, L, Q⁻, and r are the same as above, and s represents a number of 1 or more. The bonding order of the parenthesized structural units is not limited, and the bonding form thereof may be a block form or a random form. A plurality of R¹¹'s and a plurality of R¹²'s may be the same or different. The bonding order of the parenthesized structural units is not limited, and the bonding form thereof may be a block form or a random form.

In the general formulae (1-1) and (1-2), R¹¹'s are each independently preferably an alkyl group having 1 or more and 6 or less carbon atoms, or a phenyl group, more preferably a methyl group or an ethyl group, and still more preferably a methyl group.

In the general formulae (1-1) and (1-2), R¹²'s are preferably a hydrocarbon group having 1 or more and 20 or less carbon atoms or an amide bond-containing hydrocarbon group having 1 or more and 20 or less carbon atoms, and at least one of R¹²'s is more preferably an amide bond-containing hydrocarbon group having 1 or more and 20 or less carbon atoms.

In the general formulae (1-1) and (1-2), L is preferably *¹-R¹³-CH₂-CHOH-CH₂-*². Here, R¹³ is a divalent organic group, preferably an alkylene group having 1 or more and 20 or less carbon atoms or an oxyalkylene group having 1 or more and 20 or less carbon atoms, more preferably an oxyalkylene group having 1 or more and 20 or less carbon atoms, and even more preferably an oxyalkylene group having 2 or more and 6 or less carbon atoms, *¹ represents a binding moiety to the silicon atom, and *² represents a binding moiety to the nitrogen atom.

In the general formulae (1-1) and (1-2), r is preferably a number of 2 or more and 200 or less, and more preferably a number of 2 or more and 100 or less.

In the general formula (1-2), s is preferably a number of 1 or more and 50 or less, more preferably a number of 2 or more and 20 or less, and even more preferably a number of 2 or more and 10 or less.

In the general formulae (1-1) and (1-2), Q⁻ represents an anion including a halide ion such as a chloride ion or a bromide ion; and an organic acid ion such as an alkyl sulfate ion having 1 or more and 3 or less carbon atoms, an acetate ion, a lactate ion, a benzoate ion, an adipate ion, a formate ion, a malate ion, a citrate ion, or a glycolate ion. Among these, an organic acid ion is preferred, and an acetate ion or a lactate ion is more preferred.

From the viewpoint of improving an emulsion state immediately after preparation and emulsion stability, the quaternary ammonium cation-modified silicone is preferably the quaternary ammonium cation-modified silicone having a structure represented by the general formula (1-1), and more preferably at least one selected from the group consisting of a quaternary ammonium cation-modified silicone represented by the following general formula (1-1-1) and a quaternary ammonium cation-modified silicone represented by the following general formula (1-1-2).

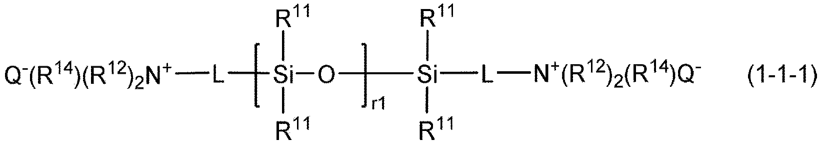

In the formula (1-1-1), each R¹¹ represents a hydrocarbon group having 1 or more and 6 or less carbon atoms, each R¹² represents a hydrogen atom, a hydrocarbon group having 1 or more and 20 or less carbon atoms, or an amide bond-containing hydrocarbon group having 1 or more and 20 or less carbon atoms, each R¹⁴ represents a hydrocarbon group having 1 or more and 20 or less carbon atoms, or an amide bond-containing hydrocarbon group having 1 or more and 20 or less carbon atoms, L represents a divalent organic group, Q⁻ is a counterion of a quaternary ammonium ion, and r1 represents a number of 2 or more. A plurality of R¹¹'s and a plurality of R¹²'s may be the same or different. The bonding order of the parenthesized structural units is not limited, and the bonding form thereof may be a block form or a random form.

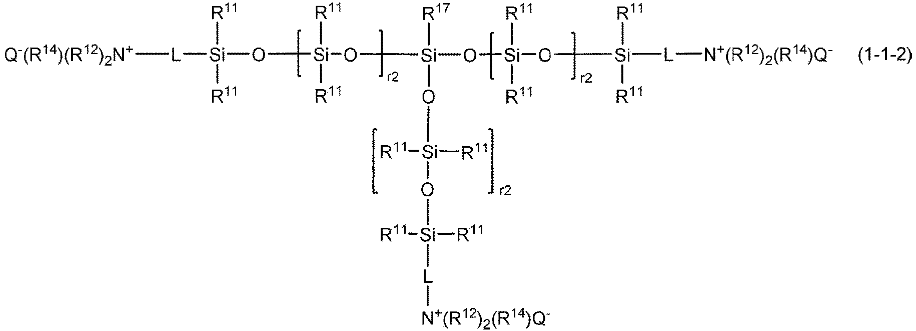

In the formula (1-1-2), R¹¹'s, R¹²'s,R¹⁴'s, L's, and Q⁻'s, are the same as above, R¹⁷ represents an alkyl group having 1 or more and 6 or less carbon atoms or a phenyl group, and r2's represent a number of 2 or more. The bonding order of the parenthesized structural units is not limited, and the bonding form thereof may be a block form or a random form.

In the general formulae (1-1-1) and (1-1-2), R¹¹'s are the same as exemplified in the general formula (1-1) and each independently preferably an alkyl group having 1 or more and 6 or less carbon atoms, or a phenyl group, more preferably a methyl group or an ethyl group, and still more preferably a methyl group.

In the general formulae (1-1-1) and (1-1-2), R¹²'s are the same as exemplified in the general formula (1-1) and preferably a hydrocarbon group having 1 or more and 20 or less carbon atoms, and more preferably a methyl group.

In the general formulae (1-1-1) and (1-1-2), L is the same as exemplified in the general formula (1-1), and preferably *¹-R¹³-CH₂-CHOH-CH₂-*². Here, R¹³ is preferably an alkylene group having 1 or more and 20 or less carbon atoms or an oxyalkylene group having 1 or more and 20 or less carbon atoms, more preferably an oxyalkylene group having 1 or more and 20 or less carbon atoms, and even more preferably an oxyalkylene group having 2 or more and 6 or less carbon atoms, *¹ represents a binding moiety to the silicon atom, and *² represents a binding moiety to the nitrogen atom.

In the general formulae (1-1-1) and (1-1-2), R¹⁴'s are preferably a hydrocarbon group having 1 or more and 20 or less carbon atoms or an amide bond-containing hydrocarbon group having 1 or more and 20 or less carbon atoms, more preferably an amide bond-containing hydrocarbon group having 1 or more and 20 or less carbon atoms, and still more preferably - R¹⁵-NHCO-R¹⁶.

Here, R¹⁵ is an alkylene group or an oxyalkylene group each having 1 or more and 18 or less carbon atoms, preferably an alkylene group having 1 or more and 18 or less carbon atoms, and more preferably an alkylene group having 2 or more and 6 or less carbon atoms, and R¹⁶ is an alkyl group having 1 or more and 18 or less carbon atoms, and preferably an alkyl group having 8 or more and 18 or less carbon atoms.

In the general formula (1-1-2), R¹⁷ is preferably an alkyl group having 1 or more and 6 or less carbon atoms or a phenyl group, and more preferably a phenyl group.

In the general formula (1-1-1), r1 is preferably a number of 2 or more and 200 or less, more preferably a number of 2 or more and 100 or less, even more preferably a number of 10 or more and 100 or less, and still even more preferably a number of 20 or more and 100 or less.

In the general formula (1-1-2), r2's are preferably a number of 2 or more and 200 or less, and more preferably a number of 2 or more and 100 or less.

In the general formulae (1-1-1) and (1-1-2), Q⁻'s are the same as exemplified in the general formula (1-1) and represents an anion including a halide ion such as a chloride ion or a bromide ion; and an organic acid ion such as an alkyl sulfate ion having 1 or more and 3 or less carbon atoms, an acetate ion, a lactate ion, a benzoate ion, an adipate ion, a formate ion, a malate ion, a citrate ion, or a glycolate ion. Among these, an organic acid ion is preferred, and an acetate ion or a lactate ion is more preferred.

The quaternary ammonium cation-modified silicone is preferably a quaternary ammonium cation-modified silicone represented by the following general formula (1-1-1') among the quaternary ammonium cation-modified silicones represented by the general formula (1-1-1), and more preferably a quaternary ammonium cation-modified silicone represented by the following general formula (1-1-2') among the quaternary ammonium cation-modified silicones represented by the general formula (1-1-2).

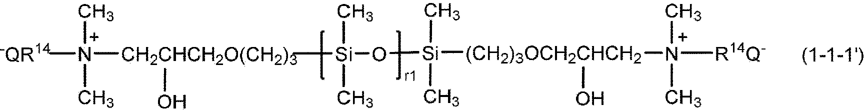

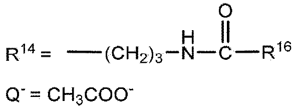

In the formula (1-1-1'), r1 is the same as above, and R¹⁶ is an alkyl group having 8 or more and 18 or less carbon atoms.

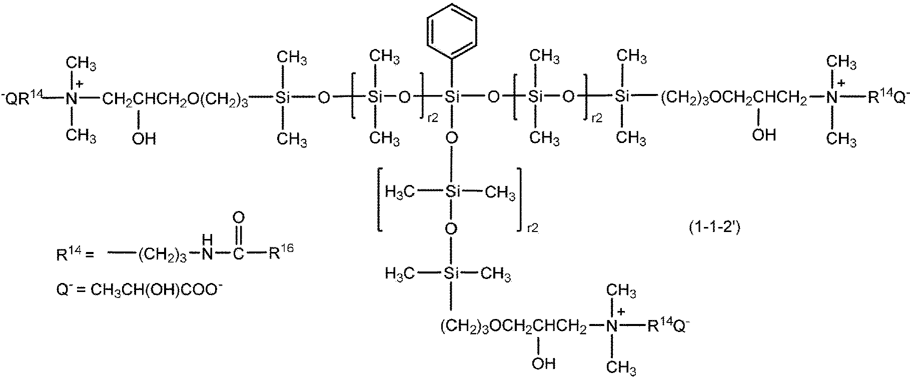

In the formula (1-1-2'), r2's are the same as above, and R¹⁶ is an alkyl group having 8 or more and 18 or less carbon atoms.

Examples of the quaternary ammonium cation-modified silicone represented by the general formula (1-1-1') include Quaternium-80. Specific examples thereof include "ABIL QUAT 3272" (r1 = 30) and "ABIL QUAT 3474" (r1 = 80) available from Evonik Industries AG.

Examples of the quaternary ammonium cation-modified silicone represented by the general formula (1-1-2') include Silicone Quaternium-22. Specific examples thereof include "ABIL T QUAT 60" and "ABIL ME 45" available from Evonik Industries AG.

Examples of the quaternary ammonium cation-modified silicone other than Quaternium-80 and Silicone Quaternium-22 include Silicone Quaternium-1, Silicone Quaternium-2, Silicone Quaternium-3, Silicone Quaternium-4, Silicone Quaternium-5, Silicone Quaternium-6, Silicone Quaternium-7, Silicone Quaternium-8, Silicone Quaternium-9, Silicone Quaternium-10, Silicone Quaternium-11, Silicone Quaternium-12, Silicone Quaternium-15, Silicone Quaternium-16, Silicone Quaternium-17, Silicone Quaternium-18, Silicone Quaternium-20, Silicone Quaternium-21, Silicone Quaternium-2 Panthenol Succinate, Silicone Quaternium-16/Glycidyl Dimethicone Crosspolymer, and Polysilicone-19.

The silicone having a quaternary ammonium group is preferably a poly(N-acylalkyleneimine)/organopolysiloxane copolymer in which a poly(N-acylalkyleneimine) segment including a repeating unit represented by the following general formula (2-1) is bound to a terminal or side chain of an organopolysiloxane segment through an alkylene group containing a hetero atom (hereinafter also simply referred to as "poly(N-acylalkyleneimine)/organopolysiloxane copolymer").

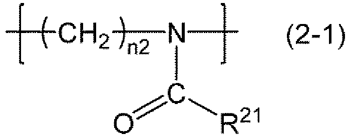

In the formula (2-1), R²¹ represents a hydrogen atom, an alkyl group having 1 or more and 22 or less carbon atoms, an aralkyl group having 7 or more and 22 or less carbon atoms, or an aryl group having 6 or more and 22 or less carbon atoms, and n2 represents 2 or 3.

A specific example of the poly(N-acylalkyleneimine)/organopolysiloxane copolymer is preferably one including a modified organopolysiloxane segment represented by the following general formula (2-2) (hereinafter also referred to as "modified organopolysiloxane segment (a)" or "segment (a)"), and a poly(N-acylalkyleneimine) segment including a repeating unit represented by the general formula (2-1) (hereinafter also referred to as "poly(N-acylalkyleneimine) segment (b)" or "segment (b)).

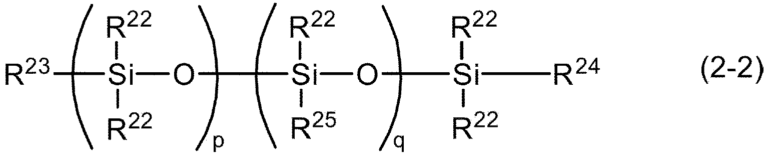

In the formula (2-2), R²²'s each independently represent an alkyl group or phenyl group having 1 or more and 22 or less carbon atoms, R²³ and R²⁴ each independently represent an alkyl group or phenyl group having 1 or more and 22 or less carbon atoms, or a divalent linking group represented by any of the following formulae (2-i) to (2-vi), R²⁵ represents a divalent linking group represented by any of the following formulae (2-i) to (2-vi), p is an integer of 2 or more and 4,000 or less, and q is an integer of 2 or more and 250 or less.

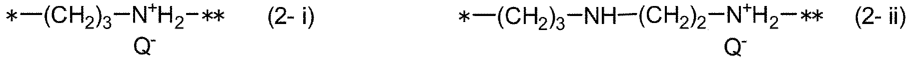

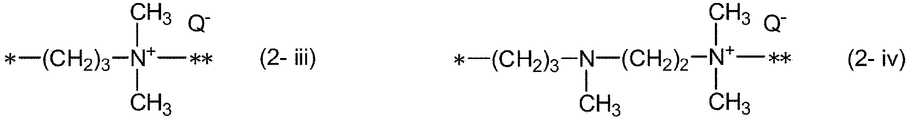

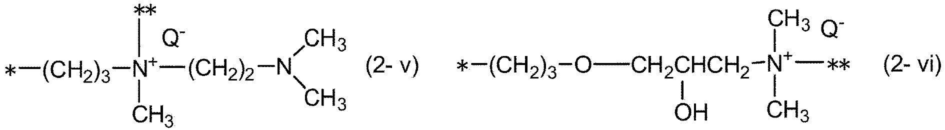

In the formulae (2-i) to (2-vi), * represents a moiety that binds to a silicon atom in the general formula (2-2), ** represents a moiety that binds to the poly(N-acylalkyleneimine) segment including a repeating unit represented by the general formula (2-1), and Q⁻ is a counterion of a quaternary ammonium ion.

Examples of the alkyl group having 1 or more and 22 or less carbon atoms represented by R²¹ in the general formula (2-1) include linear, branched, or cyclic alkyl groups having 1 or more and 22 or less carbon atoms. Specifically, examples thereof include a methyl group, an ethyl group, a n-propyl group, an isopropyl group, a n-butyl group, a tert-butyl group, a pentyl group, a hexyl group, a cyclohexyl group, a heptyl group, an octyl group, a nonyl group, a decyl group, an undecyl group, a dodecyl group, an octadecyl group, a nonadecyl group, an eicosyl group, and a docosyl group. Among these, from the viewpoint of improving an emulsion state immediately after preparation and emulsion stability, R²¹ is preferably an alkyl group having 1 or more and 10 or less carbon atoms, and more preferably an alkyl group having 1 or more and 6 or less carbon atoms. The alkyl groups are preferably a linear or branched alkyl group, and more preferably a linear alkyl group.

Examples of the aralkyl group having 7 or more and 22 or less carbon atoms represented by R²¹ in the general formula (2-1) include a benzyl group, a phenethyl group, a trityl group, a naphthylmethyl group, and an anthracenylmethyl group. Among these, from the viewpoint of improving an emulsion state immediately after preparation and emulsion stability, R²¹ is preferably an aralkyl group having 7 or more and 14 or less carbon atoms, and more preferably an aralkyl group having 7 or more and 10 or less carbon atoms.

Examples of the aryl group having 6 or more and 22 or less carbon atoms represented by R²¹ in the general formula (2-1) include a phenyl group, a tolyl group, a xylyl group, a naphthyl group, a biphenyl group, an anthryl group, and a phenanthryl group. Among these, from the viewpoint of improving an emulsion state immediately after preparation and emulsion stability, R²¹ is preferably an aryl group having 6 or more and 14 or less carbon atoms, and more preferably an aryl group having 6 or more and 12 or less carbon atoms.

In particular, from the viewpoint of improving an emulsion state immediately after preparation and emulsion stability, R²¹ in the general formula (2-1) is preferably an alkyl group having 1 or more and 22 or less carbon atoms, more preferably an alkyl group having 1 or more and 10 or less carbon atoms, even more preferably an alkyl group having 1 or more and 6 or less carbon atoms, even more preferably a methyl group or an ethyl group, and still even more preferably an ethyl group.

In the general formula (2-1), n2 is preferably 2.

Examples of the alkyl groups having 1 or more and 22 or less carbon atoms represented by R²² to R²⁴ in the general formula (2-2) include linear, branched, or cyclic alkyl groups having 1 or more and 22 or less carbon atoms. Specific examples include those described as examples of the alkyl groups having 1 or more and 22 or less carbon atoms represented by R²¹.

From the viewpoint of improving an emulsion state immediately after preparation and emulsion stability, R²²'s in the general formula (2-2) are preferably a linear or branched alkyl group having 1 or more and 10 or less carbon atoms, more preferably a linear or branched alkyl group having 1 or more and 6 or less carbon atoms, even more preferably a linear or branched alkyl group having 1 or more and 3 or less carbon atoms, and still even more preferably a methyl group.

When in the general formula (2-2), R²³ and R²⁴ represent an alkyl group or phenyl group having 1 or more and 22 or less carbon atoms, R²³ and R²⁴ are preferably a linear or branched alkyl group having 1 or more and 10 or less carbon atoms, more preferably a linear or branched alkyl group having 1 or more and 6 or less carbon atoms, even more preferably a linear or branched alkyl group having 1 or more and 3 or less carbon atoms, and still even more preferably a methyl group.

In the general formula (2-2), the divalent linking groups represented by R²³ to R²⁵ function as a linking group that links the modified organopolysiloxane segment (a) to the poly(N-acylalkyleneimine) segment (b). Among the groups of the formulae (2-i) to (2-vi), the divalent linking group is preferably the group represented by the formula (2-i) or (2-ii), and more preferably the group represented by the formula (2-i).

Examples of anions represented by Q⁻ in the formulae (2-i) to (2-vi) include an ethylsulfate ion, a methylsulfate ion, a chlorine ion, an iodine ion, a sulfate ion, a p-toluenesulfate ion, and a perchlorate ion.

In the general formula (2-2), p is preferably an integer of 135 or more and 1,600 or less, more preferably an integer of 400 or more and 1,350 or less, and even more preferably an integer of 800 or more and 1,350 or less.

In the general formula (2-2), q is preferably an integer of 3 or more and 50 or less, more preferably an integer of 5 or more and 25 or less, even more preferably an integer of 5 or more and 20 or less, and still even more preferably an integer of 5 or more and 10 or less.

From the viewpoint of improving an emulsion state immediately after preparation and emulsion stability, the poly(N-acylalkyleneimine)/organopolysiloxane copolymer is preferably at least one selected from the group consisting of a poly(N-formylethyleneimine)/organopolysiloxane copolymer, a poly(N-acetylethyleneimine)/organopolysiloxane copolymer, and a poly(N-propionylethyleneimine)/organopolysiloxane copolymer, more preferably a poly(N-propionylethyleneimine)/organopolysiloxane copolymer, and even more preferably a poly(N-propionylethyleneimine)/dimethylpolysiloxane copolymer.

As the poly(N-acylalkyleneimine)/organopolysiloxane copolymer, one or a combination of two or more may be used.

The poly(N-acylalkyleneimine)/organopolysiloxane copolymer can be produced by a publicly known method. For example, the poly(N-acylalkyleneimine)/organopolysiloxane copolymer can be obtained by a method in which a poly(N-acylalkyleneimine) that is a ring-opening polymer of a cyclic imino ether is reacted with a modified organopolysiloxane. More specifically, the poly(N-acylalkyleneimine)/organopolysiloxane copolymer can be obtained by a method described in WO2011/074585.

From the viewpoint of improving an emulsion state immediately after preparation and emulsion stability, the mass ratio (content of segment (a)/(total content of segment (a) and segment (b))) of the content of the modified organopolysiloxane segment (a) to the total content of the modified organopolysiloxane segment (a) and the poly(N-acylalkyleneimine) segment (b) in the poly(N-acylalkyleneimine)/organopolysiloxane copolymer is preferably 0.1 or more, more preferably 0.3 or more, even more preferably 0.4 or more, and still even more preferably 0.5 or more, and preferably 0.99 or less, more preferably 0.98 or less, even more preferably 0.97 or less, and still even more preferably 0.96 or less.

In this specification, the mass ratio (content of segment (a)/(total content of segment (a) and segment (b))) is the ratio of the mass (Ma) of the modified organopolysiloxane segment (a) to the total amount of the mass (Ma) of the segment (a) and the mass (Mb) of the segment (b) in the poly(N-acylalkyleneimine)/organopolysiloxane copolymer.

The mass ratio (content of segment (a)/(total content of segment (a) and segment (b))) can be calculated from the integral ratio between the alkyl group or the phenyl group in the segment (a) and the methylene group in the segment (b) by dissolving 5 mass% of the poly(N-acylalkyleneimine)/organopolysiloxane copolymer in deuterated chloroform, followed by nuclear magnetic resonance (¹H-NMR) analysis.

From the viewpoint of improving an emulsion state immediately after preparation and emulsion stability, the weight-average molecular weight (Mwsi) of the modified organopolysiloxane segment (a) is preferably 7,000 or more, more preferably 10,000 or more, even more preferably 30,000 or more, even more preferably 50,000 or more, and still even more preferably 70,000 or more, and preferably 300,000 or less, more preferably 200,000 or less, and even more preferably 130,000 or less.

Since the modified organopolysiloxane segment (a) has a skeleton common to a modified organopolysiloxane that is a raw material compound for the poly(N-acylalkyleneimine)/organopolysiloxane copolymer, the weight-average molecular weight (Mwsi) of the modified organopolysiloxane segment (a) can be considered to be equal to the weight-average molecular weight of the modified organopolysiloxane.

The weight-average molecular weight of the modified organopolysiloxane used as the raw material compound is the weight-average molecular weight determined in terms of polystyrene under the following measurement conditions by gel permeation chromatography (GPC).

### [Measurement Conditions]

Column: two columns GPC KF-804L (product name, manufactured by Showa Denko K.K.) are connected in series
Eluent: 1 mmol/L N,N-dimethyl-n-dodecylamine/chloroform
Flow rate: 1.0 mL/min
Column temperature: 40°C
Detector: differential refractive index detector (RID), 40°C
Sample concentration: 5 mg/mL
Sample injection amount: 100 µL
Standard substance: polystyrene

From the viewpoint of improving an emulsion state immediately after preparation and emulsion stability, the number-average molecular weight (Mnox) of the poly(N-acylalkyleneimine) segment (b) is preferably 500 or more, more preferably 700 or more, and even more preferably 800 or more, and preferably 10,000 or less, more preferably 7,000 or less, and even more preferably 6,000 or less.

The number-average molecular weight (Mnox) of the poly(N-acylalkyleneimine) segment (b) can be calculated from the molecular weight and polymerization degree of the N-acylalkyleneimine unit in the poly(N-acylalkyleneimine)/organopolysiloxane copolymer. Since the poly(N-acylalkyleneimine) segment (b) has a skeleton common to a poly(N-acylalkyleneimine) that is a raw material compound for the poly(N-acylalkyleneimine)/organopolysiloxane copolymer, the number-average molecular weight (Mnox) of the poly(N-acylalkyleneimine) segment (b) can be considered to be equal to the number-average molecular weight of the poly(N-acylalkyleneimine). The number-average molecular weight of the poly(N-acylalkyleneimine) can be determined by a method described in Examples through GPC.

From the viewpoint of improving an emulsion state immediately after preparation and emulsion stability, the weight-average molecular weight (Mwt) of the poly(N-acylalkyleneimine)/organopolysiloxane copolymer is preferably 10,000 or more, more preferably 30,000 or more, even more preferably 50,000 or more, and still even more preferably 60,000 or more, and preferably 500,000 or less, more preferably 300,000 or less, even more preferably 200,000 or less, even more preferably 150,000 or less, and still even more preferably 110,000 or less.

The weight-average molecular weight (Mwt) of the poly(N-acylalkyleneimine)/organopolysiloxane copolymer can be determined from the weight-average molecular weight (Mwsi) of the modified organopolysiloxane (a) in the poly(N-acylalkyleneimine)/organopolysiloxane copolymer and the mass ratio (content of segment (a)/total content of segment (a) and segment (b))).

### (Cationic Vinyl Polymer)

From the viewpoint of improving an emulsion state immediately after preparation and emulsion stability, the cationic vinyl polymer is preferably a vinyl polymer containing a structural unit derived from a monomer having at least one type of hydrophobic moiety (hereinafter also simply referred to as "hydrophobic moiety-containing monomer") selected from the group consisting of a hydrocarbon group and an alkyleneoxy group having 3 or more carbon atoms, and a structural unit derived from a monomer having a cationic group (hereinafter also simply referred to as "cationic group-containing monomer").

The "hydrophobic moiety" in the "hydrophobic moiety-containing monomer" in the present invention means a moiety having hydrophobic properties in terms of a relatively hydrophilic or hydrophobic relationship with monomers constituting the vinyl polymer, other than the hydrophobic moiety-containing monomer.

Examples of the hydrophobic moiety-containing monomer include an alkyl (meth)acrylate; aromatic group-containing monomers such as an aryl (meth)acrylate, an alkylaryl (meth)acrylate, and a styrene monomer; and a polyalkylene glycol (meth)acrylate having an alkyleneoxy group having 3 or more carbon atoms.

"(Meth)acrylate" means one or more selected from the group consisting of acrylates and methacrylates. Hereinafter, "(meth)" has the same meaning. The molecular weight of the aromatic group-containing monomer is preferably less than 500.

The structural unit derived from the hydrophobic moiety-containing monomer may be one structural unit or two or more different structural units.

The alkyl (meth)acrylate is preferably an alkyl (meth)acrylate having an alkyl group having 1 or more and 22 or less carbon atoms, preferably 1 or more and 18 or less carbon atoms, and more preferably 6 or more and 18 or less carbon atoms. Examples thereof include (meth)acrylates having a linear alkyl group, such as methyl (meth)acrylate, ethyl (meth)acrylate, propyl (meth)acrylate, butyl (meth)acrylate, pentyl (meth)acrylate, octyl (meth)acrylate, decyl (meth)acrylate, dodecyl (meth)acrylate, and stearyl (meth)acrylate; (meth)acrylates having a branched alkyl group, such as isopropyl (meth)acrylate, isobutyl (meth)acrylate, tert-butyl (meth)acrylate, isopentyl (meth)acrylate, isooctyl (meth)acrylate, isodecyl (meth)acrylate, isododecyl (meth)acrylate, isostearyl (meth)acrylate, and 2-ethylhexyl (meth)acrylate; and (meth)acrylates having an alicyclic alkyl group, such as cyclohexyl (meth)acrylate.

The aryl (meth)acrylate is preferably an aryl (meth)acrylate having an aryl group having 6 or more and 14 or less carbon atoms, and preferably 6 or more and 10 or less carbon atoms. Examples thereof include phenyl (meth)acrylate, 1-naphthyl (meth)acrylate, and 2-naphthyl (meth)acrylate.

The alkylaryl (meth)acrylate is preferably an alkylaryl (meth)acrylate having an alkylaryl group having 7 or more and 15 or less carbon atoms, and preferably 7 or more and 8 or less carbon atoms. For example, benzyl (meth)acrylate and phenylethyl (meth)acrylate are preferred, and benzyl (meth)acrylate is more preferred.

The styrene monomer is preferably styrene, α-methylstyrene, vinyltoluene, or divinylbenzene, and more preferably styrene or α-methylstyrene.

The hydrophobic moiety-containing monomer may have a functional group having a hetero atom as long as the effects of the present invention are not impaired. Examples of the functional group include alkoxy groups such as a methoxy group, an ethoxy group, a propoxy group, and a butoxy group; a phenoxy group; and a hydroxy group, which mean a moiety bound to the alkyl (meth)acrylate, the aryl (meth)acrylate, the alkylaryl (meth)acrylate, or the styrene monomer at any position.

For example, in the case of the alkyl (meth)acrylate, examples include 2-ethoxyethyl (meth)acrylate in which an ethoxy group is bound to the 2-position of the ethyl group of ethyl (meth)acrylate, 2-butoxyethyl (meth)acrylate in which a butoxy group is bound to the 2-position of the ethyl group of ethyl (meth)acrylate, 2-phenoxyethyl (meth)acrylate in which a phenoxy group is bound to the 2-position of the ethyl group of ethyl (meth)acrylate, and 2-hydroxyethyl (meth)acrylate in which a hydroxy group is bound to the 2-position of the ethyl group of ethyl (meth)acrylate.

In the case of the aryl (meth)acrylate, examples include 4-ethylphenyl (meth)acrylate in which an ethyl group is bound to the 4-position of the phenyl group of phenyl (meth)methcrylate.

In the case of the alkylaryl (meth)acrylate, examples include 4-ethylphenylmethyl (meth)acrylate in which an ethyl group is bound to the 4-position of the phenyl group of benzyl (meth)methcrylate.

When the hydrophobic moiety-containing monomer has a functional group having a hetero atom, the functional group is preferably at least one selected from the group consisting of an alkoxy group and a phenoxy group, more preferably an alkoxy group, and even more preferably at least one selected from the group consisting of a methoxy group, an ethoxy group, a propoxy group, and a butoxy group.

From the viewpoint of improving an emulsion state immediately after preparation and emulsion stability, examples of the polyalkylene glycol (meth)acrylate having an alkyleneoxy group having 3 or more carbon atoms include polypropylene glycol (m = 2 to 30, m represents a number of repeating units of the alkylene oxide, and hereinafter as the same) (meth)acrylate, octoxy (ethylene oxide-propylene oxide copolymerization) (m=2 to 30, in which the number of propylene oxide repeating units is 1 to 29) (meth)acrylate, and phenoxy (ethylene oxide-propylene oxide copolymerization) (m=2 to 30, in which the number of propylene oxide repeating units is 1 to 29) (meth)acrylate. Among these, polypropylene glycol (m=2 to 30) (meth)acrylate is preferred.

Examples of commercially available products of the polyalkylene glycol (meth)acrylate include Blemmer PP-500, Blemmer PP-800, and Blemmer PP-1000 (all available from NOF CORPORATION).

The structural unit derived from the cationic group-containing monomer is a structural unit having a cationic group represented by the following formula (3). The structural unit derived from the cationic group-containing monomer may be one structural unit or two or more different structural units.

The structural unit derived from the cationic group-containing monomer is, for example, a structural unit derived from a monomer represented by the following formula (3').

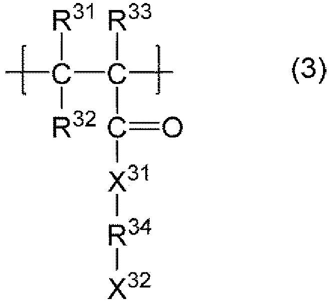

In the formula (3), R³¹ to R³³ are the same or different from each other, and each represent a hydrogen atom or an alkyl group having 1 or 2 carbon atoms, X³¹ represents an oxygen atom or a NR¹⁸ group, R¹⁸ represents a hydrogen atom or a hydrocarbon group having 1 or more and 4 or less carbon atoms, R³⁴ represents an alkylene group having 1 or more and 4 or less carbon atoms, X³² represents N⁺R³⁵R³⁶R³⁷X³³ or NR³⁸R³⁹, R³⁵ to R³⁹ are the same or different from each other, and each represent a hydrogen atom or a hydrocarbon group having 1 or more and 4 or less carbon atoms, and X³³ represents an anion.

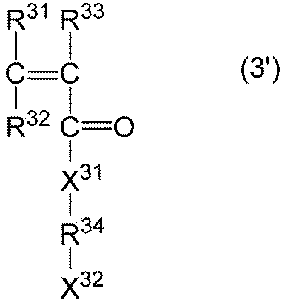

In the formula (3'), R³¹ to R³⁴, X³¹, and X³² are as described above.

From the viewpoint of improving an emulsion state immediately after preparation and emulsion stability, specific examples or preferable aspects of R³¹ to R³⁴, X³¹, and X³⁴ in the formulae (3) and (3') are as follows.

R³¹ and R³² are preferably a hydrogen atom.

R³³ is preferably a hydrogen atom or a methyl group, and more preferably a methyl group.

X³¹ is preferably an oxygen atom.

R³⁴ is preferably an alkylene group having 2 or 3 carbon atoms, and more preferably an alkylene group having 2 carbon atoms.

X³² is preferably N⁺R³⁵R³⁶R³⁷X³³, and R³⁵, R³⁶, and R³⁷ are each preferably a methyl group or an ethyl group, and more preferably a methyl group from the same viewpoint as described above.

R³⁸ and R³⁹ are preferably a methyl group or an ethyl group, and more preferably a methyl group from the viewpoint of easiness of quaternization reaction.

X³³ is preferably a halide ion or C₂H₅SO₄⁻, and more preferably a halide ion.

From the viewpoint of improving an emulsion state immediately after preparation and emulsion stability, the structural unit derived from the cationic group-containing monomer is preferably a structural unit derived from N,N-(dialkylamino)alkyl (meth)acrylate or a quaternized compound thereof, more preferably a structural unit derived from a quaternized N,N-(dialkylamino)alkyl (meth)acrylate, even more preferably a structural unit derived from at least one selected from the group consisting of quaternized 2-(dimethylamino)ethyl (meth)acrylate, quaternized 2-(diethylamino)ethyl (meth)acrylate, and quaternized 3-(dimethylamino)propyl (meth)acrylate, and still even more preferably a structural unit derived from quaternized 2-(dimethylamino)ethyl (meth)acrylate.

In this specification, "(meth)acrylic acid" means acrylic acid or methacrylic acid.

The cationic vinyl polymer may contain a structural unit other than the structural unit derived from the hydrophobic moiety-containing monomer and the structural unit derived from the cationic group-containing monomer as long as the effects of the present invention are not impaired. Examples of the other structural unit include structural units derived from nonioic monomers such as alkyl (meth)acrylamide and vinyl pyrrolidone; and other monomers such as a betaine group-containing monomer.

In this specification, "(meth)acrylamide" means acrylamide or methacrylamide.

Examples of the betaine group-containing monomer include monomers having a carbobetaine group, such as N-carboxymethyl-N-(meth)acryloyloxyethyl-N,N-dimethylammonium betaine, and N-carboxymethyl-N-(meth)acryloylamidopropyl-N,N-dimethylammonium betaine; monomers having a sulfobetaine group, such as N-(3-sulfopropyl)-N-(meth)acryloyloxyethyl-N,N-dimethylammonium betaine, and N-(3-sulfopropyl)-N-(meth)acryloylamidopropyl-N,N-dimethylammonium betaine; and monomers having a phosphobetaine group, such as 2-methacryloyloxyethylphosphorylcholine.

Examples of the cationic vinyl polymer include polyquaternium-99 (butyl methacrylate/2-ethoxyethyl methacrylate/ethyltrimethylammonium chloride methacrylate copolymer), and polyquaternium-48 (ethyl methacrylate betaine/2-hydroxyethyl methacrylate/ethyltrimethylammonium chloride methacrylate copolymer). Specific examples thereof include "PLASCIZE L-514" and "PLASCIZE L-450W" available from GOO Chemical Co., Ltd.

As described above, from the viewpoint of improving an emulsion state immediately after preparation and emulsion stability, the cationic vinyl polymer is preferably a vinyl polymer containing a structural unit derived from at least one selected from the group consisting of an alkyl (meth)acrylate optionally having a functional group having a hetero atom, an aromatic group-containing monomer optionally having a functional group having a hetero atom, and a polyalkylene glycol (meth)acrylate having an alkyleneoxy group having 3 or more carbon atoms and optionally having a functional group having a hetero atom, and a structural unit derived from N,N-(dialkylamino)alkyl (meth)acrylate or a quaternized compound thereof, more preferably a vinyl polymer containing a structural unit derived from an alkyl (meth)acrylate optionally having a functional group having a hetero atom, and a structural unit derived from N,N-(dialkylamino)alkyl (meth)acrylate or a quaternized compound thereof, even more preferably a vinyl polymer containing a structural unit derived from an alkyl (meth)acrylate having an alkyl group having 1 or more and 22 or less carbon atoms, and a structural unit derived from N,N-(dialkylamino)alkyl (meth)acrylate or a quaternized compound thereof, and still even more preferably a vinyl polymer containing a structural unit derived from an alkyl (meth)acrylate having an alkyl group having 1 or more and 18 or less carbon atoms, and a structural unit derived from N,N-(dialkylamino)alkyl (meth)acrylate or a quaternized compound thereof.

Here, in a case of havingthe functional group having a hetero atom, the functional group is preferably at least one selected from the group consisting of an alkoxy group and a phenoxy group, more preferably an alkoxy group, and even more preferably at least one selected from the group consisting of a methoxy group, an ethoxy group, a propoxy group, and a butoxy group.

### (Cationized Cellulose Derivative)

The cationized cellulose derivative is preferably a hydrophobic cationized cellulose, and more preferably at least one selected from the group consisting of a hydrophobic cationized hydroxyethyl cellulose having a long chain alkyl group, and a hydrophobic cationized hydroxypropyl cellulose having a propyleneoxy group moiety.

The hydrophobic cationized hydroxyethyl cellulose (hereinafter also referred to as "C-HEC") is preferably one having a main chain derived from anhydroglucose represented by the following general formula (4-1). 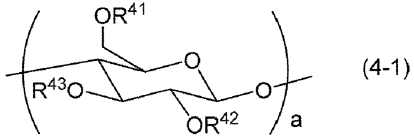 In the formula (4-1), R⁴¹, R⁴², and R⁴³ each independently represent a substituent represented by the following general formula (4-2), and a is the average polymerization degree of anhydroglucose and is 20 or more and 5.000 or less. R⁴¹, R⁴², and R⁴³ may be the same or different from each other. Plural (a) of R⁴¹'s, plural (a) of R⁴²'s, and plural (a) of R⁴³'s may be the same or different from each other. 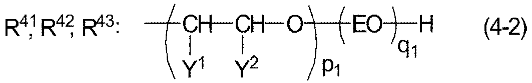 In the formula (4-2), one of Y¹ and Y² is a hydrogen atom, and the other represents a cationic group represented by the following general formula (4-3), and EO represents an ethyleneoxy group. p1 represents the number of cationized ethyleneoxy groups (-CH(Y¹)-CH(Y²)-O-) contained in the general formula (4-2), q1 represents the number of ethyleneoxy groups (-EO-) contained in the general formula (4-2), and p1 and q1 are each 0 or a positive integer. Provided that all p1 and q1 in R⁴¹, R⁴², and R⁴³ are not 0 simultaneously. When neither p1 nor q1 is 0, the order of addition of the cationized ethyleneoxy groups and the ethyleneoxy groups is not specified. When p1 and/or q1 is 2 or more, a bond may be either block bond or random bond.

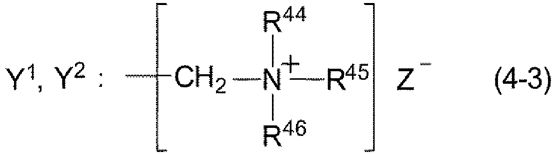

In the formula (4-3), R⁴⁴, R⁴⁵, and R⁴⁶ each independently represent a linear or branched alkyl group having 1 or more and 18 or less carbon atoms, and Z⁻ represents an anion. Provided that at least one of all R⁴⁶'s present in the molecule is a linear or branched alkyl group having 8 or more and 18 or less carbon atoms.

Examples of R⁴⁴, R⁴⁵, and R⁴⁶ in the general formula (4-3) include a methyl group, an ethyl group, a n-propyl group, a n-butyl group, a n-octyl group, a n-decyl group, a n-dodecyl group, a n-tetradecyl group, a n-hexadecyl group, and a n-octadecyl group.

R⁴⁴ and R⁴⁵ are preferably at least one selected from the group consisting of a methyl group, an ethyl group, a n-propyl group, a n-butyl group, a n-octyl group, a n-decyl group, and a n-dodecyl group, and more preferably a methyl group.

R⁴⁶ is preferably at least one selected from the group consisting of a methyl group, a n-octyl group, a n-decyl group, and a n-dodecyl group, and more preferably a n-dodecyl group.

In the general formula (4-3), Z⁻ represents an anion that is a counterion of an ammonium cation. Z⁻ is not particularly limited as long as it is an anion. Specific examples thereof include an alkylsulfate ion, a sulfate ion, a phosphate ion, an alkylcarbonate ion, and a halide ion. Among these, from the viewpoint of easy production, a halide ion is preferred. Examples of the halide ion include a fluoride ion, a chloride ion, a bromide ion, and an iodide ion. From the viewpoint of chemical stability, a chloride ion or a bromide ion is preferred, and a chloride ion is more preferred.

The degree of substitution of a cationized ethyleneoxy group in C-HEC is preferably 0.01 or more, and preferably 3.0 or less from the viewpoint of improving an emulsion state immediately after preparation and emulsion stability and from the viewpoint of easy production.

In the present invention, the "degree of substitution of a cationized ethyleneoxy group" refers to the average amount by mole of cationized ethyleneoxy group present in the molecule of C-HPC per mole of anhydroglucose unit (hereinafter also referred to as "AGU") constituting a cellulose main chain. The degree of substitution of a cationized ethyleneoxy group is determined, specifically, by the same method as a method for determining the degree of substitution of a cationized ethyleneoxy group described in WO2011/059063.

The degree of substitution of an ethyleneoxy group in C-HEC is preferably 0.5 or more, and preferably 4.0 or less from the viewpoint of improving an emulsion state immediately after preparation and emulsion stability and from the viewpoint of easy production.

In the present invention, the "degree of substitution of an ethyleneoxy group" refers to the average amount by mole of ethyleneoxy group present in the molecule of C-HEC per mole of AGU constituting a cellulose main chain. The degree of substitution of an ethyleneoxy group is determined in accordance with a method for determining the degree of substitution of a propyleneoxy group described in WO2011/059063.

Examples of such C-HEC include polyquaternium-67. Specific examples thereof include "SoftCAT SL-100", "SoftCAT SL-5", "SoftCAT SL-30", and "SoftCAT SL-60" available from Dow Chemical Company.

Furthermore, examples of C-HEC other than polyquaternium-67 include a polymer of a quaternary ammonium salt (polyquaternium-24) obtained by addition polymerization of glycidyllauryldimethylammonium chloride to hydroxyethyl cellulose, and a polymer of a quaternary ammonium salt (polyquaternium-72) obtained by addition reaction of coconut oil alkyldimethylammonium-substituted epoxide to hydroxyethyl cellulose.

The hydrophobic cationized hydroxypropyl cellulose (hereinafter also referred to as "C-HPC") is preferably one having a main chain derived from anhydroglucose represented by the following general formula (4-4).

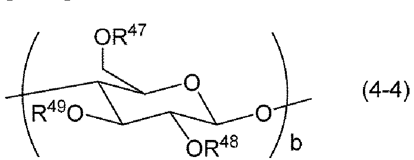

In the formula (4-4), R⁴⁷, R⁴⁸, and R⁴⁹ each independently represent a substituent represented by the following general formula (4-5), and b is the average polymerization degree of anhydroglucose and is 20 or more and 5.000 or less. R⁴⁷, R⁴⁸, and R⁴⁹ may be the same or different from each other. Plural (b) of R⁴⁷'s, plural (b) of R⁴⁸'s, and plural (b) of R⁴⁹'s may be the same or different from each other.

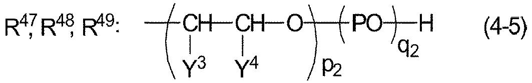

In the formula (4-5), one of Y³ and Y⁴ is a hydrogen atom, and the other represents a cationic group represented by the following general formula (4-6), and PO represents a propyleneoxy group. p2 represents the number of cationized ethyleneoxy groups (-CH(Y³)-CH(Y⁴)-O-) contained in the general formula (4-5), q2 represents the number of propyleneoxy groups (-PO-) contained in the general formula (4-5), and p2 and q2 are each 0 or a positive integer. Provided that all p2 and q2 in R⁴⁷, R⁴⁸, and R⁴⁹ are not 0 simultaneously. When neither p2 nor q2 is 0, the order of addition of the cationized ethyleneoxy groups and the propyleneoxy groups is not specified. When p2 and/or q2 is 2 or more, a bond may be either block bond or random bond.

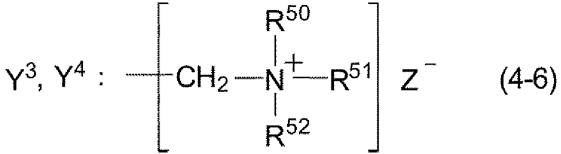

In the formula (4-6), R⁵⁰, R⁵¹, and R⁵² each independently represent a linear or branched alkyl group having 1 or more and 3 or less carbon atoms, and Z⁻ represents an anion.

From the viewpoint of improving an emulsion state immediately after preparation and emulsion stability, the average degree of polymerization b in the general formula (4-4) is preferably 20 or more, more preferably 50 or more, and even more preferably 100 or more. From the same viewpoint as described above and from the viewpoint of easy production, the average degree of polymerization b in the general formula (4-4) is preferably 5,000 or less, more preferably 3,000 or less, and even more preferably 2,000 or less.

The "average degree of polymerization" in the present invention refers to the average viscometric degree of polymerization measured by a copper-ammonia method, and specifically calculated by a method described in WO2011/059063.

p2 represents the number of cationized ethyleneoxy groups (-CH(Y³)-CH(Y⁴)-O-) contained in the general formula (4-5), and is 0 or a positive integer. From the viewpoint of easy production, p2 is preferably an integer of 0 or more and 3 or less, more preferably an integer of 0 or more and 2 or less, and even more preferably 0 or 1.

q2 represents the number of propyleneoxy groups (-PO-) contained in the general formula (4-5), and is 0 or a positive integer. From the viewpoint of easy production, q2 is preferably an integer of 0 or more and 4 or less, more preferably an integer of 0 or more and 2 or less, and even more preferably 0 or 1.

When there are a plurality of substituents represented by the general formula (4-5) in the molecule of C-HPC, values of p2 in the substituents may be different, and values of q2 in the substituents may be different.

From the viewpoint of easy production, a sum of p2 and q2 is preferably an integer of 1 or more and 5 or less, more preferably an integer of 1 or more and 4 or less, even more preferably an integer of 1 or more and 3 or less, and still even more preferably 1 or 2.

When neither p2 nor q2 is 0, the order of addition of the cationized ethyleneoxy groups and the propyleneoxy groups is not specified, and from the viewpoint of easy production, the order described in the general formula (4-5) is preferred.

When neither p2 nor q2 is 0 and p2 and/or q2 is 2 or more, a bond may be either block bond or random bond, and from the viewpoint of easy production, a block bond is preferred.

In at least one of plural (b) of R⁴⁷'s, plural (b) of R⁴⁸'s, and plural (b) of R⁴⁹'s, p2 in the general formula (4-5) is not 0, or in at least one thereof, q2 in the general formula (4-5) is not 0.

In the general formula (4-5), R⁴⁷, R⁴⁸, and R⁴⁹ are each independently a linear or branched alkyl group having 1 or more and 3 or less carbon atoms, and specific examples thereof include a methyl group, an ethyl group, a n-propyl group, and an isopropyl group. Among these, a methyl group or an ethyl group is preferred, and a methyl group is more preferred.

In the general formula (4-6), Z⁻ represents an anion that is a counter-ion of an ammonium cation. Z⁻ is not particularly limited as long as it is an anion. Specific examples thereof include an alkylsulfate ion, a sulfate ion, a phosphate ion, an alkylcarbonate ion, and a halide ion. Among these, from the viewpoint of easy production, a halide ion is preferred. Examples of the halide ion include a fluoride ion, a chloride ion, a bromide ion, and an iodide ion. From the viewpoint of chemical stability, a chloride ion or a bromide ion is preferred, and a chloride ion is more preferred.

The degree of substitution of a cationized ethyleneoxy group in C-HPC is preferably 0.01 or more, and preferably 2.9 or less from the viewpoint of improving an emulsion state immediately after preparation and emulsion stability and from the viewpoint of easy production.

In the present invention, the "degree of substitution of a cationized ethyleneoxy group" refers to the average amount by mole of cationized ethyleneoxy group present in the molecule of C-HPC per mole of anhydroglucose unit (AGU) constituting a cellulose main chain. The degree of substitution of a cationized ethyleneoxy group is determined, specifically, by the method described in WO2011/059063.

The degree of substitution of a propyleneoxy group in C-HPC is preferably 0.1 or more, and preferably 4.0 or less from the viewpoint of improving an emulsion state immediately after preparation and emulsion stability and from the viewpoint of easy production.

In the present invention, the "degree of substitution of a propyleneoxy group" refers to the average amount by mole of propyleneoxy groups present in the molecule of C-HPC per mole of AGU constituting a cellulose main chain. The degree of substitution of a propyleneoxy group is determined, specifically, by the method described in WO2011/059063.

As described above, from the viewpoint of improving an emulsion state immediately after preparation and emulsion stability, the component (B1) is preferably at least one selected from the group consisting of a silicone having at least one quaternary ammonium group at an end or side chain of organopolysiloxane, a vinyl polymer containing a structural unit derived from a monomer having at least one type of hydrophobic moiety selected from the group consisting of a hydrocarbon group and an alkyleneoxy group having 3 or more carbon atoms, and a structural unit derived from a monomer having a cationic group, hydrophobic cationized hydroxyethyl cellulose having a long chain alkyl group, and a hydrophobic cationized hydroxypropyl cellulose having a propyleneoxy moiety, more preferably at least one selected from the group consisting of a silicone having at least one quaternary ammonium group at an end or side chain of organopolysiloxane, and a vinyl polymer containing a structural unit derived from a monomer having at least one type of hydrophobic moiety selected from the group consisting of a hydrocarbon group and an alkyleneoxy group having 3 or more carbon atoms, and a structural unit derived from a monomer having a cationic group, even more preferably at least one selected from the group consisting of a poly(N-acylalkyleneimine)/organopolysiloxane copolymer in which a poly(N-acylalkyleneimine) segment including a repeating unit represented by the following general formula (2-1) is bound to an end or side chain of an organopolysiloxane segment through an alkylene group containing a hetero atom, and a vinyl polymer containing a structural unit derived from an alkyl (meth)acrylate optionally having a functional group having a hetero atom, and a structural unit derived from N,N-(dialkylamino)alkyl (meth)acrylate or a quaternized compound thereof, even more preferably at least one selected from the group consisting of a poly(N-acylalkyleneimine)/organopolysiloxane copolymer in which a poly(N-acylalkyleneimine) segment including a repeating unit represented by the following general formula (2-1) is bound to an end or side chain of an organopolysiloxane segment through an alkylene group containing a hetero atom, and a vinyl polymer containing a structural unit derived from an alkyl (meth)acrylate having an alkyl group having 1 or more and 22 or less carbon atoms, and a structural unit derived from N,N-(dialkylamino)alkyl (meth)acrylate or a quaternized compound thereof, and still even more preferably at least one selected from the group consisting of a poly(N-acylalkyleneimine)/organopolysiloxane copolymer in which a poly(N-acylalkyleneimine) segment including a repeating unit represented by the following general formula (2-1) is bound to an end or side chain of an organopolysiloxane segment through an alkylene group containing a hetero atom, and a vinyl polymer containing a structural unit derived from an alkyl (meth)acrylate having an alkyl group having 1 or more and 18 or less carbon atoms, and a structural unit derived from N,N-(dialkylamino)alkyl (meth)acrylate or a quaternized compound thereof.

Here, when there is a functional group having a hetero atom, the functional group is preferably at least one selected from the group consisting of an alkoxy group and a phenoxy group, more preferably an alkoxy group, and even more preferably at least one selected from the group consisting of a methoxy group, an ethoxy group, a propoxy group, and a butoxy group.

### [(B2) Cationic Surfactant]

From the viewpoint of improving an emulsion state immediately after preparation and emulsion stability, the carbon number of the alkyl group of (B2) the cationic surfactant (hereinafter also referred to as "component (B2)") is preferably 7 or more, more preferably 8 or more, even more preferably 10 or more, even more preferably 12 or more, and still even more preferably 14 or more, and preferably 22 or less.

The alkyl group in the component (B2) is preferably at least one selected from the group consisting of an aliphatic alkyl group and an aromatic alkyl group, and more preferably an aliphatic alkyl group.

The aliphatic alkyl group may be either a linear alkyl group or a branched alkyl group.

Examples of the aromatic alkyl group include a benzyl group.

As the component (B2), one or two or more can be used.

Examples of the component (B2) include (i) an alkyltrimethylammonium salt, (ii) an alkoxyalkyltrimethylammonium salt, (iii) a dialkyldimethylammonium salt, (iv) an alkylamidoalkyltrimethylammonium salt, (v) an alkyldimethylamine and its salt, (vi) an alkoxyalkyldimethylamine and its salt, (vii) an alkylamidoalkyldimethylamine and its salt, and (viii) a monoalkylbenzyldimethylammonium salt.

Examples of (i) the alkyltrimethylammonium salt include an alkyltrimethylammonium salt having an alkyl group having preferably 12 or more and 22 or less carbon atoms, and more preferably 16 or more and 20 or less carbon atoms. Specifically, examples thereof include cetyltrimethylammonium chloride (cetrimonium chloride), stearyltrimethylammonium chloride (steartrimonium chloride), and behenyltrimethylammonium chloride (behentrimonium chloride).

Examples of (ii) the alkoxyalkyltrimethylammonium salt include an alkoxyalkyltrimethylammonium salt having an alkoxy group having preferably 12 or more and 22 or less carbon atoms, and more preferably 16 or more and 20 or less carbon atoms. Specifically, examples thereof include stearoxypropyltrimethylammonium chloride, stearoxyethyltrimethylammonium chloride, and stearoxyhydroxypropyltrimethylammonium chloride.

Examples of (iii) the dialkyldimethylammonium salt include a dialkyldimethylammonium salt having an alkyl group having preferably 12 or more and 22 or less carbon atoms, and more preferably 16 or more and 20 or less carbon atoms. Specifically, examples thereof include dicetyldimethylammonium chloride (dicetyldimonium chloride), distearyldimethylammonium chloride (di steary dimonium chloride), and dipalmitorylethyldimethylammonium chloride (dipalmitoylethydimonium chloride).

Examples of (iv) the alkylamidoalkyltrimethylammonium salt include an alkylamidoalkyltrimethylammonium salt having an alkyl group having preferably 11 or more and 21 or less carbon atoms, and more preferably 13 or more and 19 or less carbon atoms. Specifically, examples thereof include palmitamidopropyltrimethylammonium chloride (palmitamidopropyltrimonium chloride).

Each of (v) the alkyldimethylamine, (vi) the alkoxyalkyldimethylamine, and (vii) the alkylamidoalkyldimethylamine reacts with an acid to become a tertiary amine salt and works as a cationic surfactant.

The alkyl group of each of (v) the alkyldimethylamine and its salt and (vi) the alkoxyalkyldimethylamine and its salt is an alkyl group having preferably 12 or more and 22 or less carbon atoms, and more preferably 16 or more and 20 or less carbon atoms.

The alkyl group of (vii) the alkylamidoalkyldimethylamine and its salt is an alkyl group having preferably 11 or more and 21 or less carbon atoms, and more preferably 15 or more and 19 or less carbon atoms.

Examples of (viii) the monoalkylbenzyldimethylammonium salt include a monoalkylbenzyldimethylammonium salt having an alkyl group having preferably 8 or more and 18 or less carbon atoms. Specifically, examples thereof include benzalkonium chloride.

The amine in each of (v) to (vii) may be reacted with an acid to form a salt in advance and the salt may be blended in an external preparation for skin; or the amine may be blended as it is in the external preparation for skin, with which an acid is then blended to form a salt in the composition. Therefore, the amine and its salt are herein defined as the cationic surfactant. The content or blending amount thereof is expressed in terms of the mass of the amine.

Examples of the salts of the amines in (v) to (vii) include salts of an organic acid or an inorganic acid. Examples of the organic acid include monocarboxylic acids, such as acetic acid and propionic acid; dicarboxylic acids, such as malonic acid, succinic acid, glutaric acid, adipic acid, maleic acid, fumaric acid, and phthalic acid; polycarboxylic acids, such as polyglutamic acid; hydroxycarboxylic acids, such as glycolic acid, lactic acid, hydroxyacrylic acid, glyceric acid, malic acid, tartaric acid, and citric acid; and acidic amino acids, such as glutamic acid and aspartic acid. Examples of the inorganic acid include hydrochloric acid, sulfuric acid, and phosphoric acid. Among these, an organic acid is preferred, and at least one selected from the group consisting of a dicarboxylic acid, a hydroxycarboxylic acid, and an acidic amino acid is more preferred. Among dicarboxylic acids, at least one selected from the group consisting of maleic acid and succinic acid is more preferred. Among hydroxycarboxylic acids, at least one selected from the group consisting of glycolic acid, lactic acid, and malic acid is more preferred. Among acidic amino acids, glutamic acid is more preferred.

Examples of (v) the alkyldimethylamine and its salt include N,N-dimethylbehenylamine, N,N-dimethylstearylamine, and their organic acid salts, and lactic acid salt of N,N-dimethylbehenylamine, a glycolic acid salt of N,N-dimethylstearylamine, and the like are preferred.

Examples of (vi) the alkoxyalkyldimethylamine and its salt include N,N-dimethyl-3-hexadecyloxypropylamine, N,N-dimethyl-3-octadecyloxypropylamine, and their organic acid salts, and N,N-dimethyl-3-hexadecyloxypropylamine or its salt, N,N-dimethyl-3-octadecyloxypropylamine [stearoxypropyldimethylamine] or its salt, and N,N-dimethyl-3-octadecyloxypropylamine or its salt are preferred.

Examples of (vii) the alkylamidoalkyldimethylamine and its salt include N-[3-(dimethylamino)propyl]docosanamide, N-[3-(dimethylamino)propyl]stearamide, and their organic acid salts, and a lactic acid salt of N-[3-(dimethylamino)propyl]docosanamide and a glycolic acid salt of N-[3-(dimethylamino)propyl]stearamide are preferred.

Among these, from the viewpoint of improving an emulsion state immediately after preparation and emulsion stability, the component (B2) is preferably a cationic surfactant having an alkyl group having 8 or more and 22 or less carbon atoms, more preferably at least one selected from the group consisting of (i) the alkyltrimethylammonium salt, (ii) the alkoxyalkyltrimethylammonium salt, (iii) the dialkyldimethylammonium salt, (v) the alkyldimethylamine and its salt, (vi) the alkoxyalkyldimethylamine and its salt, and (vii) the alkylamidoalkyldimethylamine and its salt, even more preferably at least one selected from the group consisting of (i) the alkyltrimethylammonium salt, and (iii) the dialkyldimethylammonium salt, even more preferably (iii) the dialkyldimethylammonium salt, even more preferably the dialkyldimethylammonium salt having an alkyl group having 12 or more and 22 or less carbon atoms, and still even more preferably the dialkyldimethylammonium salt having an alkyl group having 16 or more and 20 or less carbon atoms.

From the viewpoint of improving an emulsion state immediately after preparation and emulsion stability, the content of the component (B) in the external preparation for skin of the present invention is preferably 0.01 mass% or more, more preferably 0.02 mass% or more, and even more preferably 0.03 mass% or more, and preferably 1 mass% or less, more preferably 0.7 mass% or less, even more preferably 0.5 mass% or less, and still even more preferably 0.3 mass% or less. More specifically, from the viewpoint of improving an emulsion state immediately after preparation and emulsion stability, the content of the component (B) in the external preparation for skin of the present invention is preferably 0.01 to 1 mass%, more preferably 0.02 to 0.7 mass%, even more preferably 0.02 to 0.5 mass%, even more preferably 0.02 to 0.3 mass%, and still even more preferably 0.03 to 0.3 mass%.

From the viewpoint of improving an emulsion state immediately after preparation and emulsion stability, the mass ratio (component (B)/component (A)) of the content of the component (B) to the content of the component (A) in the external preparation for skin of the present invention is preferably 0.003 or more, more preferably 0.005 or more, and even more preferably 0.007 or more, and preferably 0.30 or less, more preferably 0.20 or less, even more preferably 0.10 or less, and still even more preferably 0.05 or less. More specifically, the mass ratio (component (B)/component (A)) is preferably 0.003 to 0.30, more preferably 0.005 to 0.20, even more preferably 0.005 to 0.10, even more preferably 0.005 to 0.05, and still even more preferably 0.007 to 0.05.

From the viewpoint of improving an emulsion state immediately after preparation and emulsion stability, the content of the component (B1) in the external preparation for skin of the present invention is preferably 0.01 mass% or more, more preferably 0.02 mass% or more, and even more preferably 0.03 mass% or more, and preferably 1 mass% or less, more preferably 0.7 mass% or less, even more preferably 0.5 mass% or less, and still even more preferably 0.3 mass% or less. More specifically, from the viewpoint of improving an emulsion state immediately after preparation and emulsion stability, the content of the component (B1) in the external preparation for skin of the present invention is preferably 0.01 to 1 mass%, more preferably 0.02 to 0.7 mass%, even more preferably 0.02 to 0.5 mass%, even more preferably 0.02 to 0.3 mass%, and still even more preferably 0.03 to 0.3 mass%.

From the viewpoint of improving an emulsion state immediately after preparation and emulsion stability, the mass ratio (component (B1)/component (A)) of the content of the component (B1) to the content of the component (A) in the external preparation for skin of the present invention is preferably 0.003 or more, more preferably 0.005 or more, and even more preferably 0.007 or more, and preferably 0.30 or less, more preferably 0.20 or less, even more preferably 0.10 or less, and still even more preferably 0.05 or less. More specifically, from the viewpoint of improving an emulsion state immediately after preparation and emulsion stability, the mass ratio (component (B1)/component (A)) is preferably 0.003 to 0.30, more preferably 0.005 to 0.20, even more preferably 0.005 to 0.10, even more preferably 0.005 to 0.05, and still even more preferably 0.007 to 0.05.

From the viewpoint of improving an emulsion state immediately after preparation and emulsion stability, the content of the component (B2) in the external preparation for skin of the present invention is preferably 0.01 mass% or more, more preferably 0.02 mass% or more, even more preferably 0.03 mass% or more, even more preferably 0.04 mass% or more, even more preferably 0.05 mass% or more, and still even more preferably 0.1 mass% or more, and preferably 1 mass% or less, more preferably 0.7 mass% or less, and even more preferably 0.5 mass% or less. More specifically, from the viewpoint of improving an emulsion state immediately after preparation and emulsion stability, the content of the component (B2) in the external preparation for skin of the present invention is preferably 0.01 to 1 mass%, more preferably 0.02 to 1 mass%, even more preferably 0.03 to 1 mass%, even more preferably 0.04 to 1 mass%, even more preferably 0.05 to 1 mass%, even more preferably 0.05 to 0.7 mass%, and still even more preferably 0.1 to 0.5 mass%.

From the viewpoint of improving an emulsion state immediately after preparation and emulsion stability, the mass ratio (component (B2)/component (A)) of the content of the component (B2) to the content of the component (A) in the external preparation for skin of the present invention is preferably 0.005 or more, more preferably 0.01 or more, even more preferably 0.03 or more, and still even more preferably 0.05 or more, and preferably 0.30 or less, more preferably 0.20 or less, even more preferably 0.15 or less, and still even more preferably 0.10 or less. More specifically, from the viewpoint of improving an emulsion state immediately after preparation and emulsion stability, the mass ratio (component (B2)/component (A)) is preferably 0.005 to 0.30, more preferably 0.01 to 0.30, even more preferably 0.01 to 0.20, even more preferably 0.03 to 0.20, even more preferably 0.05 to 0.15, and still even more preferably 0.05 to 0.10.

### <Component (C)>

The external preparation for skin of the present invention contains as a component (C) an oily component.

In the present invention, the "oily component" means a component having a solubility in water of 1 w/w% or less. Examples of the component (C) include an ultraviolet absorber, an oil agent, an emulsifier, and a dispersant.

### [Ultraviolet Absorber]

From the viewpoint of improving an ultraviolet protection effect, the component (C) preferably contains an ultraviolet absorber. The ultraviolet absorber is preferably at least one selected from the group consisting of a liquid organic ultraviolet absorber and a solid organic ultraviolet absorber.

In this specification, a "liquid (form)" refers to a state that exhibits flowability in an environment at 1 atmospheric pressure and 25°C, that is, a state under a temperature condition that is equal to or higher than a melting point (in an amorphous substance having no melting point, a state under a temperature condition that is equal to or higher than a melt-point). A "solid (form)" refers to a state that does not exhibit flowability in an environment at 1 atmospheric pressure and 25°C, that is, a state under a temperature condition that is lower than a melting point (in an amorphous substance having no melting point, a state under a temperature condition that is lower than a melt-point).

As the ultraviolet absorber, one may be contained alone, or two or more may be contained as appropriate in combination.

Examples of the liquid organic ultraviolet absorber include cinnamate-based ultraviolet absorbers such as 2-ethylhexyl p-methoxycinnamate (ethylhexyl methoxycinnamate), 2-ethoxyethyl p-methoxycinnamate, a mixture of isopropyl p-methoxycinnamate and diisopropylcinnamate ester, and methylbis(trimethylsiloxy)silylisopentyl trimethoxycinnamate; p-dimethylaminobenzate-based ultraviolet absorbers such as amyl p-dimethylaminobenzoate and 2-ethylhexyl p-dimethylaminobenzoate; salicylate-based ultraviolet absorbers such as ethylene glycol salicylate, ethylhexyl salicylate, butyloctyl salicylate, benzyl salicylate, and homomenthyl salicylate (homosalate); octocrylene; ethylhexyl methoxycrylene; and dimethyldiethyl benzalmalonate.

Examples of the solid organic ultraviolet absorber include 2-(4-diethylamino-2-hydroxybenzoyl) benzoic acid hexyl ester (hexyl diethylaminohydroxybenzoylbenzoate), 2,4-bis{ [4-(2-ethylhexyloxy)-2-hydroxy]phenyl}-6-(4-methoxyphenyl)-1,3,5-triazine (bis-ethylhexyloxyphenol methoxyphenyl triazine), 2,4,6-tris[4-(2-ethylhexyloxycarbonyl)anilino]-1,3,5-triazine (ethylhexyl triazone), 4-tert-butyl-4'-methoxydibenzoylmethane (t-butyl methoxydibenzoylmethane), and 2-ethylhexyl dimethoxybenzylidene dioxoimidazolidine propionate.

Examples of commercially available products of the ultraviolet absorber include "UVINUL MC80" (2-ethylhexyl p-methoxycinnamate), "UVINUL A PLUS GRANULAR" (2-(4-diethylamino-2-hydroxybenzoyl) benzoic acid hexyl ester), "TINOSORB S" (2,4-bis{[4-(2-ethylhexyloxy)-2-hydroxy]phenyl}-6-(4-methoxyphenyl)-1,3,5-triazine), "UVINUL T-150" (2,4,6-tris[4-(2-ethylhexyloxycarbonyl)anilino]-1,3,5-triazine) (all available from BASF); "Softshade DH" (2-ethylhexyl dimethoxybenzylidene dioxoimidazolidine propionate) (available from Ajinomoto Co., Inc.); "PARSOL 1789" (4-tert-butyl-4'-methoxydibenzoylmethane), and PARSOL SLX (polysilicone-15 (dimethyldiethyl benzalmalonate) (all available from DSM).

From the viewpoint of improving an UV protection effect, the content of the ultraviolet absorber in the external preparation for skin of the present invention is preferably 3 mass% or more, more preferably 5 mass% or more, and even more preferably 10 mass% or more, and preferably 30 mass% or less, more preferably 25 mass% or less, even more preferably 20 mass% or less, and still even more preferably 15 mass% or less. More specifically, from the viewpoint of improving an UV protection effect, the content of the ultraviolet absorber in the external preparation for skin of the present invention is preferably 3 to 30 mass%, more preferably 5 to 25 mass%, even more preferably 5 to 20 mass%, and still even more preferably 10 to 15 mass%.

### [Liquid Oil Agent]

From the viewpoint of improving an emulsion state immediately after preparation and emulsion stability, the component (C) may contain an oily component other than the ultraviolet absorber. From the viewpoint of imparting an emollient effect to a skin, the component (C) preferably contains the oily component other than the ultraviolet absorber. Examples of the oily component include an oil agent in a liquid form in an environment at 1 atmospheric pressure and 25°C.

Examples of the liquid oil agent include oil agents used for typical cosmetics. Among these, the liquid oil agent is preferably at least one selected from the group consisting of an ester oil, a hydrocarbon oil, a silicone oil, a higher alcohol, and a higher fatty acid. As the liquid oil agent, one may be contained alone, or two or more may be contained as appropriate in combination.

Examples of the ester oil include a synthetic ester oil and a natural oil and fat.

Examples of the ester oil include esters of monovalent carboxylic acids having 2 or more and 26 or less carbon atoms with monohydric alcohols having 1 or more and 30 or less carbon atoms, esters of monobasic carboxylic acids having 2 or more and 26 or less carbon atoms with polyhydric alcohols having 2 or more and 10 or less carbon atoms, and esters of polybasic carboxylic acids having 2 or more and 18 or less carbon atoms with monohydric alcohols having 1 or more and 30 or less carbon atoms.

Among these, from the viewpoint of improving an emulsion state immediately after preparation and emulsion stability, the ester oil is preferably at least one selected from the group consisting of a monoester of a fatty acid having 4 or more and 18 or less carbon atoms with an alcohol having 2 or more and 24 or less carbon atoms, a diester of a fatty acid having 6 or more and 18 or less carbon atoms with a dialcohol having 2 or more and 10 or less carbon atoms, a triester of a fatty acid having 6 or more and 18 or less carbon atoms with glycerin, an ester of a fatty acid having 6 or more and 18 or less carbon atoms with pentaerythritol, a diester of a dicarboxylic acid having 2 or more and 18 or less carbon atoms with a branched alcohol having 2 or more and 18 or less carbon atoms, and alkyl benzoate.

The monoester of a fatty acid having 4 or more and 18 or less carbon atoms with an alcohol having 2 or more and 24 or less carbon atoms is preferably at least one selected from the group consisting of isodecyl neopentanoate, isononyl isononanoate, isotridecyl isononanoate, isopropyl myristate, isocetyl myristate, octyldodecyl myristate, isopropyl palmitate, ethylhexyl palmitate, and 2-hexyldecyl palmitate.

Examples of the diester of a fatty acid having 6 or more and 18 or less carbon atoms with a dialcohol having 2 or more and 10 or less carbon atoms include diethylene glycol dicaprate, neopentyl glycol dicaprate, and neopentyl glycol di(2-ethylhexanoate).

The triester of a fatty acid having 6 or more and 18 or less carbon atoms with glycerin is preferably at least one selected from the group consisting of glyceryl tri(2-ethylhexanoate), caprylic/capric triglyceride, and a natural oil and fat.

Examples of the natural oil and fat include a castor oil, a mink oil, an avocado oil, an olive oil, a sunflower oil, a camellia oil, an apricot kernel oil, an almond oil, a wheat germ oil, a grape seed oil, a babassu oil, a jojoba oil, a macadamia seed oil, a camellia oleifera oil, and a meadowfoam oil.

The ester of a fatty acid having 6 or more and 18 or less carbon atoms with pentaerythritol is preferably at least one selected from the group consisting of dipentaerythrityl pentaisostearate, dipentaerythrityl tetraisostearate, and dipentaerythrityl tri-polyhydroxystearate.

The diester of a dicarboxylic acid having 2 or more and 18 or less carbon atoms with a branched alcohol having 2 or more and 18 or less carbon atoms is preferably at least one selected from the group consisting of diisostearyl malate, di(2-ethylhexyl) sebacate, and diisopropyl sebacate.

Examples of the alkyl benzoate include (C12-C15) alkyl benzoate (e.g., Finsolv TN; available from Innospec Active Chemicals LLC).

Specific examples of the hydrocarbon oil include isohexadecane, liquid paraffin, hydrogenated polyisobutene (liquid isoparaffin, heavy liquid isoparaffin), cycloparaffin, liquid ozokerite, scualene, scualane, pristane, α-olefin oligomer, and polybutene.

The silicone oil is preferably at least one selected from the group consisting of dimethyl silicone (dimethyl polysiloxane and dimethicon) and modified silicone, more preferably at least one selected from the group consisting of dimethyl polysiloxane and phenyl-modified silicone, and even more preferably at least one selected from the group consisting of dimethyl polysiloxane and phenyl-modified silicone that have a kinematic viscosity at 25°C of 5,000 mm²/s or less.

Examples of phenyl-modified silicone include phenyl trimethicone, diphenyl dimethicone, diphenylsiloxy phenyl trimethicone, and phenyl methicone.

The kinematic viscosity at 25°C of the silicone oil can be measured with an Ubbelohde viscometer in accordance with ASTM D 445-46T or JIS Z 8803.

Examples of commercially available products of the silicone oil include "KF-96A-6cs" (dimethyl polysiloxane), "KF-96A-10cs" (dimethyl polysiloxane), "KF-96A-20cs" (dimethyl polysiloxane), and "KF-96A-100cs" (dimethyl polysiloxane) available from Shin-Etsu Chemical Co., Ltd.; and "DOWSIL 556 COSMETIC GRADE FLUID" (phenyl trimethicone) available from Dow Chemical Company.

Examples of the higher alcohol include higher alcohols having 12 or more and 24 or less carbon atoms. Specific examples of the higher alcohol include 2-octyldodecanol, isostearyl alcohol, and oleyl alcohol.

Examples of the higher fatty acid include fatty acids having 12 or more and 22 or less carbon atoms. Specific examples of the higher fatty acid include oleic acid, isostearic acid, linoleic acid, and linolenic acid.

From the viewpoint of improving an emulsion state immediately after preparation and emulsion stability, the liquid oil agent preferably contains at least one selected from the group consisting of an ester oil, a silicone oil, and a hydrocarbon oil, more preferably contains at least one selected from the group consisting of an ester oil and a hydrocarbon oil, even more preferably contains an ester oil, and even more preferably contains at least one selected from the group consisting of a monoester of a fatty acid having 4 or more and 18 or less carbon atoms with an alcohol having 2 or more and 24 or less carbon atoms, a diester of a fatty acid having 6 or more and 18 or less carbon atoms with a dialcohol having 2 or more and 10 or less carbon atoms, a triester of a fatty acid having 6 or more and 18 or less carbon atoms with glycerin, an ester of a fatty acid having 6 or more and 18 or less carbon atoms with pentaerythritol, a diester of a dicarboxylic acid having 2 or more and 18 or less carbon atoms with a branched alcohol having 2 or more and 18 or less carbon atoms, and (C12-C15) alkyl benzoate.

From the viewpoint of improving an emulsion state immediately after preparation and emulsion stability, the content of the liquid oil agent in the external preparation for skin of the present invention is preferably 1 mass% or more, more preferably 2 mass% or more, and even more preferably 3 mass% or more, and preferably 30 mass% or less, more preferably 25 mass% or less, even more preferably 20 mass% or less, and still even more preferably 10 mass% or less. More specifically, from the viewpoint of improving an emulsion state immediately after preparation and emulsion stability, the content of the liquid oil agent in the external preparation for skin of the present invention is preferably 1 to 30 mass%, more preferably 1 to 25 mass%, even more preferably 2 to 25 mass%, even more preferably 2 to 20 mass%, and still even more preferably 3 to 10 mass%.

When the liquid oil agent contains an ester oil and a hydrocarbon oil, from the viewpoint of improving an emulsion state immediately after preparation and emulsion stability, the total content of the ester oil and the hydrocarbon oil in the liquid oil agent is preferably 60 mass% or more, more preferably 70 mass% or more, even more preferably 80 mass% or more, even more preferably 90 mass% or more, and still even more preferably 95 mass% or more, and the upper limit thereof is 100 mass%. More specifically, from the viewpoint of improving an emulsion state immediately after preparation and emulsion stability, the total content of the ester oil and the hydrocarbon oil in the liquid oil agent is preferably 60 to 100 mass%, more preferably 70 to 100 mass%, even more preferably 80 to 100 mass%, even more preferably 90 to 100 mass%, and still even more preferably 95 to 100 mass%.

When the liquid oil agent contains an ester oil, from the viewpoint of improving an emulsion state immediately after preparation and emulsion stability, the content of the ester oil in the liquid oil agent is preferably 40 mass% or more, more preferably 50 mass% or more, even more preferably 60 mass% or more, even more preferably 70 mass% or more, even more preferably 80 mass% or more, even more preferably 90 mass% or more, and still even more preferably 95 mass% or more, and the upper limit thereof is 100 mass%. More specifically, from the viewpoint of improving an emulsion state immediately after preparation and emulsion stability, the content of the ester oil in the liquid oil agent is preferably 40 to 100 mass%, more preferably 50 to 100 mass%, even more preferably 60 to 100 mass%, even more preferably 70 to 100 mass%, even more preferably 80 to 100 mass%, even more preferably 90 to 100 mass%, and still even more preferably 95 to 100 mass%.

From the viewpoint of improving an emulsion state immediately after preparation and emulsion stability, the content of a silicone oil in the liquid oil agent is preferably 20 mass% or less, more preferably 17 mass% or less, even more preferably 15 mass% or less, even more preferably 12 mass% or less, even more preferably 10 mass% or less, even more preferably 5 mass% or less, even more preferably 3 mass% or less, even more preferably 1 mass% or less, and still even more preferably 0 mass%. More specifically, from the viewpoint of improving an emulsion state immediately after preparation and emulsion stability, the content of the silicone oil in the liquid oil agent is preferably 0 to 20 mass%, more preferably 0 to 17 mass%, even more preferably 0 to 15 mass%, even more preferably 0 to 12 mass%, even more preferably 0 to 10 mass%, even more preferably 0 to 5 mass%, even more preferably 0 to 3 mass%, even more preferably 0 to 1 mass%, and still even more preferably 0 mass%.

In the present invention, from the viewpoint of emulsion stability, a solid oil agent in an environment at 1 atmospheric pressure and 25°C may be contained as the other oily component.

Examples of the solid oil agent include solid ester oils such as glyceryl monostearate, and glyceryl monomyristate; solid silicone oils such as an alkyl-modified silicone; solid hydrocarbon oils such as vaseline; solid higher alcohols such as cetyl alcohol, stearyl alcohol, and behenyl alcohol; and solid higher fatty acids such as lauric acid, myristic acid, palmitic acid, stearic acid, behenic acid, 12-hydroxystearic acid, and lanolin fatty acid.

From the viewpoint of improving an emulsion state immediately after preparation and emulsion stability, the content of the solid oil agent in the external preparation for skin of the present invention is preferably 0.1 mass% or more, more preferably 0.2 mass% or more, and even more preferably 0.3 mass% or more, and preferably 3 mass% or less, more preferably 2 mass% or less, and even more preferably 1 mass% or less.

From the viewpoint of improving an emulsion state immediately after preparation and emulsion stability, the content of the component (C) in the external preparation for skin of the present invention is preferably 5 mass% or more, more preferably 10 mass% or more, and even more preferably 15 mass% or more, and preferably 40 mass% or less, more preferably 30 mass% or less, even more preferably 25 mass% or less, and still even more preferably 20 mass% or less. More specifically, from the viewpoint of improving an emulsion state immediately after preparation and emulsion stability, the content of the component (C) in the external preparation for skin of the present invention is preferably 5 to 40 mass%, more preferably 10 to 30 mass%, even more preferably 10 to 25 mass%, and still even more preferably 15 to 20 mass%.

The component (C) preferably contains at least one selected from the group consisting of an ultraviolet absorber and an ester oil. In this case, from the viewpoint of improving an emulsion state immediately after preparation and emulsion stability, the total content of the ultraviolet absorber and the ester oil in the external preparation for skin of the present invention is preferably 5 mass% or more, more preferably 10 mass% or more, and even more preferably 15 mass% or more, and preferably 30 mass% or less, more preferably 25 mass% or less, and even more preferably 20 mass% or less. More specifically, from the viewpoint of improving an emulsion state immediately after preparation and emulsion stability, the total content of the ultraviolet absorber and the ester oil in the external preparation for skin of the present invention is preferably 5 to 30 mass%, more preferably 10 to 25 mass%, and even more preferably 15 to 20 mass%.

From the viewpoint of improving an emulsion state immediately after preparation and emulsion stability, the content of a silicone oil in the component (C) is preferably 10 mass% or less, more preferably 7 mass% or less, even more preferably 5 mass% or less, even more preferably 3 mass% or less, even more preferably 1 mass% or less, and still even more preferably 0 mass%. More specifically, from the viewpoint of improving an emulsion state immediately after preparation and emulsion stability, the content of the silicone oil in the component (C) is preferably 0 to 10 mass% , more preferably 0 to 7 mass%, even more preferably 0 to 5 mass%, even more preferably 0 to 3 mass%, even more preferably 0 to 1 mass%, and still even more preferably 0 mass%.

### <Component (D)>

The external preparation for skin of the present invention contains as a component (D) an aqueous component.

In the present invention, the "aqueous component" means a component having a solubility in water of more than 1 w/w%. Examples of the component (D) include water and an organic solvent that is optionally miscible with water (hereinafter also referred to as "water-soluble organic solvent").

The component (D) preferably contains at least one of water and the water-soluble organic solvent.

Examples of the water-soluble organic solvent include saturated monohydric alcohols having 1 or more and 3 or less carbon atoms such as ethanol and isopropyl alcohol; polyhydric alcohols such as ethylene glycol, diethylene glycol, triethylene glycol, polyethylene glycol (average molecular weight: less than 650), propylene glycol, dipropylene glycol, polypropylene glycol (average molecular weight: less than 650), isopropylene glycol, 1,3-butylene glycol, glycerin, diglycerin, and polyglycerin. As the water-soluble organic solvent, one may be used alone, or two or more may be used in combination.

Among these, the water-soluble organic solvent is preferably at least one selected from the group consisting of a saturated monohydric alcohol having 1 or more and 3 or less carbon atoms and a polyhydric alcohol, more preferably at least one selected from the group consisting of ethanol, isopropyl alcohol, ethylene glycol, diethylene glycol, propylene glycol, dipropylene glycol, 1,3-butylene glycol, and glycerin, and even more preferably at least one selected from the group consisting of ethanol, dipropylene glycol, 1,3-butylene glycol, and glycerin.

From the viewpoint of improving an emulsion state immediately after preparation and emulsion stability, the content of the component (D) in the external preparation for skin of the present invention is preferably 53 mass% or more, more preferably 55 mass% or more, and even more preferably 57 mass% or more, and preferably 80 mass% or less, more preferably 75 mass% or less, and even more preferably 73 mass% or less. More specifically, from the viewpoint of improving an emulsion state immediately after preparation and emulsion stability, the content of the component (D) in the external preparation for skin of the present invention is preferably 53 to 80 mass%, more preferably 55 to 75 mass%, and even more preferably 57 to 73 mass%.

### <Component (E)>

The external preparation for skin of the present invention preferably further contains (E) a thickener (hereinafter also referred to as "component (E)") from the viewpoint of achieving favorable emulsion stability.

The component (E) is preferably a water-soluble macromolecule having a tackiness effect, which is not particularly limited as long as it can be used for typical cosmetics. Any of a natural macromolecule, a semisynthetic macromolecule, and a synthetic macromolecule, other than the component (B 1), can be used. As the component (E), one may be used alone, or two or more may be used in combination.

Examples of the natural macromolecule include xanthan gum, carrageenan, and an alginic acid. Among these, from the viewpoint of achieving favorable emulsion stability, xanthan gum is preferred.

Examples of the semisynthetic macromolecule include modified polysaccharides such as hydroxy cellulose, hydroxyethyl cellulose, hydroxypropyl cellulose, carboxymethyl cellulose sodium, methyl cellulose, and hydroxymethyl cellulose.

Examples of the synthetic macromolecule include a vinyl polymer containing a structural unit derived from a (meth)acrylic acid or a structural unit derived from a derivative of a (meth)acrylic acid; a polyvinyl pyrrolidone, a polyvinyl alcohol, and a cationized polyvinyl pyrrolidone. Among these, from the viewpoint of achieving favorable emulsion stability, a vinyl polymer containing a structural unit derived from a (meth)acrylic acid or a structural unit derived from a derivative of a (meth)acrylic acid is preferred.

Examples of the vinyl polymer containing a structural unit derived from a (meth)acrylic acid or a structural unit derived from a derivative of a (meth)acrylic acid include a crosslinked polyacrylic acid (carbomer, carboxyvinyl polymer), a polyacrylic acid, an acrylic acid/alkyl methacrylate copolymer, polyacrylamide, polyacryloldimethyl tauric acid, a copolymer containing a structural unit derived from at least one type of acrylamide-based monomer selected from the group consisting of acrylamide and acryloyldimethyl tauric acid (hereinafter also referred to as "AMPS"), and their salts.

Examples of the copolymer containing a structural unit derived from at least one type of acrylamide-based monomer selected from the group consisting of acrylamide and AMPS include an acrylic acid/acrylamide copolymer, an acrylic acid/AMPS copolymer, an acrylamide/AMPS copolymer, an acrylic acid/acrylamide/AMPS copolymer, an acrylic acid/AMPS/dimethylacrylamide copolymer, a hydroxyethyl acrylate/AMPS copolymer, an AMPS/vinyl pyrrolidone copolymer, an AMPS/vinylformamide copolymer, an AMPS/polyoxyethylene alkyl ester of methacrylic acid (average number of moles added of ethylene oxide: 10 to 30) copolymer, and their salts.

Specific examples of the copolymers and their salts include a hydroxyethyl acrylate/acryloyldimethyl taurine Na copolymer (INCI name: Hydroxyethyl Acrylate/Sodium Acryloyldimethyl Taurate Copolymer), an acrylic acid Na/ acryloyldimethyl taurine Na copolymer (INCI name: Sodium Acrylate/Sodium Acryloyldimethyl Taurate Copolymer), an acrylic acid Na/acryloyldimethyl taurine/dimethylacrylamide crosspolymer (INCI name: Sodium Acrylate/Acryloyldimethyltaurate/Dimethylacrylamide Crosspolymer), an ammonium acryloyldimethyl taurine/vinylpyrrolidone copolymer (INCI name: Ammonium Acryloyldimethyltaurate/VP Copolymer), an acrylic acid/acrylamide/acrylic acid Na/ acryloyldimethyl taurine Na copolymer (INCI name: Polyacrylate-13), an ammonium acryloyldimethyl taurine/dimethylacrylamide/lauryl methacrylate/laureth-4 methacrylate (INCI name: Polyacrylate Crosspolymer-6), an ammonium acryloyldimethyl taurine/beheneth-25 methacrylate crosspolymer (INCI name: Ammonium Acryloyldimethyltaurate/Beheneth-25 Methacrylate Crosspolymer), and an ammonium acryloyldimethyl taurine/steareth-25 methacrylate crosspolymer (INCI name: Ammonium Acryloyldimethyltaurate/Steareth-25 Methacrylate Crosspolymer).

Among these, from the viewpoint of achieving favorable emulsion stability, the component (E) preferably contains one or more selected from the group consisting of the natural macromolecule, the semisynthetic macromolecule, and the synthetic macromolecule, more preferably contains at least one selected from the group consisting of xanthan gum, hydroxyethyl cellulose, and the vinyl polymer containing a structural unit derived from a (meth)acrylic acid or a structural unit derived from a derivative of a (meth)acrylic acid, even more preferably contains xanthan gum, and the vinyl polymer containing a structural unit derived from a (meth)acrylic acid or a structural unit derived from a derivative of a (meth)acrylic acid, even more preferably contains at least one selected from the group consisting of xanthan gum, and the vinyl polymer containing a structural unit derived from at least one type of acrylamide-based monomer selected from the group consisting of acrylamide and AMPS and their salt, and still even more preferably contains at least one selected from the group consisting of xanthan gum, polyacrylamide, polyacryloyl dimethyl tauric acid, and the copolymer containing a structural unit derived from at least one acrylamide-based monomer selected from the group consisting of acrylamide and AMPS, and their salts.

Examples of commercially available products of hydroxyethyl cellulose include "HEC Daicel SE400", "HEC SE550", "HEC SE600", "HEC SE850", and "HEC Daicel SE900" (all available from Daicel Miraizu Ltd.).

Examples of commercially available products of carbomer include "Carbopol 910", "Carbopol 934", "Carbopol 940", "Carbopol 941", "Carbopol 980", and "Carbopol 981" (all available from Lubrizol Advanced Materials, Inc.).

Examples of commercially available products of the acrylic acid/methacrylate copolymer include "Carbopol 1382", "Carbopol ETD2020", "PEMULEN TR-1", and "PEMULEN TR-2" (all available from Lubrizol Advanced Materials, Inc.).

Examples of commercially available products of the vinyl polymer containing a structural unit derived from a (meth)acrylic acid or a structural unit derived from a derivative of a (meth)acrylic acid include "SEPIGEL 501" (polyacrylamide, Polysorbate 85, mineral oil, isoparaffin), "SEPIGEL 305" (polyacrylamide, hydrogenated polyisobutene (or (C13, 14) isoparaffin), laureth-7, water), "SIMULGEL EG" (an acrylic acid Na/ acryloyldimethyl taurine Na copolymer, isohexadecane, Polysorbate 80), "SEPIMAX ZEN" (Polyacrylate Crosspolymer-6 (an ammonium acryloyldimethyl taurine/dimethylacrylamide/lauryl methacrylate/laureth-4 methacrylate), "SEPINOV EMT 10" (a hydroxyethyl acrylate/acryloyldimethyl taurine Na copolymer, sorbitan isostearate, Polysorbate 60), "SIMULGEL NS" (a hydroxyethyl acrylate/acryloyldimethyl taurine Na copolymer, scualane, Polysorbate 60, water), "SIMULGEL FL" (a hydroxyethyl acrylate/acryloyldimethyl taurine Na copolymer, isohexadecane, Polysorbate 60, water), "SEPIPLUS S" (a hydroxyethyl acrylate/acryloyldimethyl taurine Na copolymer, polyisobutene, PEG-7 trimethylolpropane coconut alkyl ether, water), "SEPIPLUS 400" (an acrylic acid/acrylamide/acrylic acid Na/ acryloyldimethyl taurine Na copolymer, polyisobutene, Polysorbate 20, water), and "SEPILIFE NUDE" (an acrylic acid Na/acryloyldimethyl taurine Na copolymer, water, (C15-19) alkane, polyglyceryl-6 laurate, polyglycerin-6, sorbitan oleate, sorbitan isostearate) (all available from SEPPIC); "Aristoflex AVC" (an ammonium acryloyldimethyltaurate/vinylpyrrolidone copolymer), "Aristoflex HMB" (an ammonium acryloyldimethyl taurine/beheneth-25 methacrylate crosspolymer), and "Aristoflex HMS" (an ammonium acryloyldimethyl taurine/steareth-25 methacrylate crosspolymer) (all available from Clariant Ltd.); and "VOLAREST FL" (an acrylate/beheneth-25 methacrylate copolymer, water, laureth sulfate Na) (available from Croda).

From the viewpoint of achieving favorable emulsion stability, the content of the component (E) in the external preparation for skin of the present invention is preferably 0.05 mass% or more, more preferably 0.10 mass% or more, and even more preferably 0.13 mass% or more, and preferably 3 mass% or less, more preferably 2 mass% or less, and even more preferably 1.5 mass% or less. More specifically, from the viewpoint of achieving favorable emulsion stability, the content of the component (E) in the external preparation for skin of the present invention is preferably 0.05 to 3 mass%, more preferably 0.10 to 2 mass%, and even more preferably 0.13 to 1.5 mass%.

From the viewpoint of improving an emulsion state immediately after preparation and emulsion stability, the mass ratio (component (E)/component (A)) of the content of the component (E) to the content of the component (A) in the external preparation for skin of the present invention is preferably 0.01 or more, more preferably 0.03 or more, and even more preferably 0.05 or more, and preferably 1.5 or less, more preferably 1.3 or less, even more preferably 0.7 or less, and still even more preferably 0.5 or less. More specifically, the mass ratio (component (B)/component (A)) is preferably 0.01 to 1.5, more preferably 0.03 to 1.3, even more preferably 0.03 to 0.7, even more preferably 0.03 to 0.5, and still even more preferably 0.05 to 0.5.

In addition to the components (A) to (E), the external preparation for skin of the present invention may appropriately contain an optional component used in the application of an external preparation for skin as long as the effects of the present invention are not impaired. Examples of the optional component include an ultraviolet scattering agent, a surfactant, a neutralizer, a pH adjustor, a bactericide, an anti-inflammatory agent, an antiseptic, a colorant, a chelating reagent, a whitening agent, an antiperspirant, an antioxidant, and a perfume, other than the components (A) to (E).

From the viewpoint of scattering ultraviolet light to enhance an ultraviolet protection effect, the external preparation for skin of the present invention may preferably contain an ultraviolet scattering agent.

The ultraviolet scattering agent is preferably inorganic particles, and more preferably metal oxide fine particles. As the ultraviolet scattering agent, one may be used alone, or two or more may be used in combination.

From the viewpoint of scattering ultraviolet light to enhance an ultraviolet protection effect, the metal oxide fine particles used as the ultraviolet scattering agent may preferably be at least one selected from the group consisting of zinc oxide fine particles, titanium oxide fine particles, and cerium oxide fine particles, and more preferably at least one selected from the group consisting of zinc oxide fine particles and titanium oxide fine particles. The metal oxide fine particles may contain divalent or higher metal, and may incorporate a metal such as iron, zirconium, calcium, manganese, magnesium, or yttrium, or an oxide thereof alone or appropriately incorporate a combination of two or more thereof.

Examples of the shape of the metal oxide fine particles include spherical, flake, rod, spindle, needle, and amorphous shapes.

From the viewpoint of enhancing an ultraviolet protection effect, the average primary particle diameter of the metal oxide fine particles is preferably less than 100 nm, more preferably 90 nm or less, even more preferably 70 nm or less, and even more preferably 50 nm or less, and preferably 1 nm or more, more preferably 3 nm or more, and even more preferably 5 nm or more.

The average primary particle diameter of the metal oxide fine particles can be determined from an observation image with a transmission electron microscope (TEM). Specifically, the average primary particle diameter can be determined by observing the metal oxide fine particles under conditions with TEM at an observation magnification of 50,000, measuring the maximum shorter diameters of 300 primary particles in the observation image, and calculating the number-average value. Here, the maximum shorter diameter means a shorter diameter that is the longest among shorter diameters orthogonal to longer diameters when the metal oxide fine particles have a shape other than the flake shape. When the metal oxide fine particles have a flake shape, the average primary particle diameter thereof can be determined by measuring the thicknesses of 300 primary particles in the observation image under the same observation conditions as described above, and calculating the number-average value.

From the viewpoint of enhancing dispersibility in the external preparation for skin to improve an ultraviolet protection effect, the metal oxide fine particles are preferably hydrophobilized metal oxide fine particles. In the present invention, the "hydrophobilized metal oxide fine particles" refer to metal oxide fine particles having a hydrophobilized surface.

Examples of hydrophobilization with an ultraviolet scattering agent include a treatment with a silicone; a treatment with an alkylalkoxysilane; a treatment with a fatty acid; a treatment with a fluorine-containing compound such as a perfluoroalkylphosphate ester or a perfluoroalcohol; a treatment with an amino acid such as N-acylglutamate; a treatment with lecithin; a treatment with a metallic soap; a treatment with an alkylphosphate ester; and an ASI treatment with a metal salt of N-acylamino acid (sodium lauroyl aspartate), zinc chloride, and alkoxy titanium alkylate (isopropyl titanium triisostearate).

One of the hydrophobization treatments may be performed alone, or two or more thereof may be combined.

Among these, from the viewpoint of enhancing dispersibility in the external preparation for skin to improve an ultraviolet protection effect, the hydrophobization treatment is preferably at least one selected from the group consisting of a treatment with a silicone, a treatment with an alkylalkoxysilane, and a treatment with a fatty acid.

Examples of a surface treatment agent used in the treatment with a silicone include various silicone oils such as methyl polysiloxane, dimethyl polysiloxane, methylphenyl polysiloxane, methylhydrogen polysiloxane, methyl cyclopolysiloxane, dodecamethylcyclohexasiloxane, tetradecamethylhexasiloxane, a dimethylsiloxane/methyl(polyoxyethylene)siloxane/methyl(polyoxypropylene)siloxane copolymer, a dimethylsiloxane/methyl(polyoxyethylene)siloxane copolymer, a dimethylsiloxane/methyl(polyoxypropylene)siloxane copolymer, a dimethylsiloxane/methylcetyloxysiloxane copolymer, a dimethylsiloxane/methylstearoxysiloxane copolymer, and an alkyl acrylate/dimethicone copolymer. As the surface treatment agent used in the treatment with a silicone, one may be used alone, or two or more may be combined. Among these, from the viewpoint of enhancing dispersibility in the external preparation for skin to improve an ultraviolet protection effect, the surface treatment agent used in the treatment with a silicone is preferably at least one selected from the group consisting of methylhydrogen polysiloxane and dimethyl polysiloxane.

From the viewpoint of enhancing dispersibility in the external preparation for skin to improve an ultraviolet protection effect, a surface treatment agent used in the treatment with an alkylalkoxysilane preferably an alkylalkoxysilane including linear or branched alkyl group having 6 or more and 20 or less carbon atoms, and more preferably at least one selected from the group consisting of octyltriethoxysilane and octyltrimethoxysilane.

Examples of a surface treatment agent used in the treatment with a fatty acid include linear or branched higher fatty acid having 12 or more and 22 or less carbon atoms. Among these, from the viewpoint of enhancing dispersibility in the external preparation for skin to improve an ultraviolet protection effect, the surface treatment agent used in the treatment with a fatty acid is preferably linear or branched higher fatty acid having 14 or more and 22 or less carbon atoms, more preferably a linear or branched higher fatty acid having 16 or more and 20 or less carbon atoms, and even more preferably at least one selected from the group consisting of stearic acid and isostearic acid.

From the viewpoint of enhancing dispersibility in the external preparation for skin to improve an ultraviolet protection effect, the amount of hydrophobization in the hydrophobized metal oxide fine particles is preferably 0.1 mass% or more, and preferably 40 mass% or less, and more preferably 30 mass% or less in the hydrophobized metal oxide fine particles.

In the present invention, when the metal oxide fine particles are those hydrophobilized, the mass and average primary particle diameter of the metal oxide fine particles mean the mass and average primary particle diameter of the metal oxide fine particles containing the surface treatment agent.

Examples of commercially available products of the hydrophobized zinc oxide fine particles include FINEX series (available from Sakai Chemical Industry Co., Ltd.), and MZ series and MZY series (all available from Tayca Corporation).

Examples of commercially available products of the hydrophobized titanium oxide fine particles include STR series (available from Sakai Chemical Industry Co., Ltd.), TTO-55 series and TTO-51 series (all available from ISHIHARA SANGYO KAISHA, LTD.), and MT series and MTY series (all available from Tayca Corporation).

From the viewpoint of enhancing an ultraviolet protection effect, the content of the ultraviolet scattering agent in the external preparation for skin of the present invention is preferably 1 mass% or more, more preferably 5 mass% or more, and even more preferably 7 mass% or more, and preferably 30 mass% or less, more preferably 25 mass% or less, even more preferably 20 mass% or less, and still even more preferably 17 mass% or less. More specifically, from the viewpoint of enhancing an ultraviolet protection effect, the content of the ultraviolet scattering agent in the external preparation for skin of the present invention is preferably 1 to 30 mass%, more preferably 5 to 25 mass%, even more preferably 5 to 20 mass%, and still even more preferably 7 to 17 mass%.

The external preparation for skin of the present invention is preferably used as a skin cosmetic. Examples of the skin cosmetic include a skin toner, a cream, a milky lotion, a cosmetic fluid, a suntan cosmetic, a base cosmetic, and a foundation. The external preparation for skin containing an ultraviolet absorber or an ultraviolet scattering agent among these is preferably adopted in applications of sunscreen as a cosmetic for sunscreen (skin toner, cream, milky lotion, and cosmetic fluid), a suntan cosmetic, a base cosmetic, a foundation, or the like since it is excellent in an ultraviolet protection effect.

The applicable dosage form of the external preparation for skin of the present invention is a liquid, foam, paste, cream, or solid form, or the like. The external preparation for skin is applicable to a sheet agent, a spray agent, or a mousse agent.

A method for applying the external preparation for skin of the present invention to a skin can be a publicly known application method according to the use form or purpose of the external preparation. Here, "applying to a skin" includes not only directly applying the external preparation to a skin surface by hand or the like but also attaching the external preparation to a skin surface by spraying or the like. The external preparation having a liquid, foam, paste, cream, or solid form can be usually applied as it is or used by spraying or the like.

### [Method for Producing External Preparation for Skin of Oil-In-Water Type Emulsification]

A method for producing the external preparation for skin of the present invention is not particularly limited, and a publicly known method can be appropriately used according to the dosage form of the external preparation for skin. Examples thereof include a method including steps of blending the components (A), (B), (C), and (D), and if necessary, the component (E) and the aforementioned optional component, and uniformly mixing the components by a homomixer or the like.

From the viewpoint of rapidly forming a stable oil-in-water type emulsion system to improve an emulsion state immediately after preparation and emulsion stability, the method for producing the external preparation for skin of the present invention is preferably a method including a step 1 of mixing the components (A) and (B) in water. In the step 1, the component (B) can be adsorbed to the surface of the component (A) to form amphiphilic silica particles.

The method for producing the external preparation for skin of the present invention is preferably a method further including, after a mixture in which the components (A) and (B) are mixed in water in the step 1 is obtained, a step of mixing and emulsifying the mixture, and a water phase component and an oil phase component, and more preferably a method including the following steps I to IV or a method including the following steps I' to IV'.

Step I: a step of adding the component (A) to the component (D), then adding the component (B) diluted with the component (D), mixing the components, and adsorbing the component (B) to the surface of the component (A), to obtain a mixture of the components (A) and (B)

Step II: mixing the component (D) and if necessary, the component (E) and the aforementioned optional water-soluble component, to obtain a water phase component

Step III: mixing the component (C) and if necessary, the aforementioned optional oil-soluble component, to obtain an oil phase component

Step IV: adding the mixture of the components (A) and (B) obtained in the step I and the oil phase component obtained in the step III to the water phase component obtained in the step II, followed by mixing and emulsifying, to obtain the external preparation for skin

Step I': a step of adding the component (A) to the component (D), then adding the component (B) diluted with the component (D), mixing the components, and adsorbing the component (B) to the surface of the component (A), to obtain a mixture of the components (A) and (B)

Step II': mixing the component (D) and if necessary, the component (E) and the aforementioned optional water-soluble component in the mixture obtained in the step I', to obtain a water phase component

Step III': mixing the component (C) and if necessary, the aforementioned optional oil-soluble component, to obtain an oil phase component

Step IV': adding the oil phase component obtained in the step III' to the water phase component obtained in the step II', followed by mixing and emulsifying, to obtain the external preparation for skin

From the viewpoint of rapidly forming a stable oil-in-water type emulsion system by forming amphiphilic silica particles to improve an emulsion state immediately after preparation and emulsion stability, the component (D) used in each of the step I and the step I' preferably contains at least water.

The mixing in the steps I, II, and IV and the mixing in the step I', II', and IV' are each preferably carried out at a temperature range of 15°C or higher and 35°C or lower.

The mixing the oil phase component in the step III and the step III' is preferably carried out by stirring under heating at a temperature range of 60°C or higher and 90°C or lower.

In the method for producing the external preparation for skin of the present invention, from the viewpoint of improving an emulsion state immediately after preparation and emulsion stability, the mass ratio (component (B)/component (A)) of the amount of the blended component (B) to the amount of the blended component (A) is preferably 0.003 or more, more preferably 0.005 or more, and even more preferably 0.007 or more, and preferably 0.30 or less, more preferably 0.20 or less, even more preferably 0.10 or less, and still even more preferably 0.05 or less. More specifically, the mass ratio (component (B)/component (A)) is preferably 0.003 to 0.30, more preferably 0.005 to 0.20, even more preferably 0.005 to 0.10, even more preferably 0.005 to 0.05, and still even more preferably 0.007 to 0.05.

In the method for producing the external preparation for skin of the present invention, from the viewpoint of improving an emulsion state immediately after preparation and emulsion stability, the mass ratio (component (B1)/component (A)) of the amount of the blended component (B1) to the amount of the blended component (A) is preferably 0.003 or more, more preferably 0.005 or more, and even more preferably 0.007 or more, and preferably 0.30 or less, more preferably 0.20 or less, even more preferably 0.10 or less, and still even more preferably 0.05 or less. More specifically, from the viewpoint of improving an emulsion state immediately after preparation and emulsion stability, the mass ratio (component (B1)/component (A)) is preferably 0.003 to 0.30, more preferably 0.005 to 0.20, even more preferably 0.005 to 0.10, even more preferably 0.005 to 0.05, and still even more preferably 0.007 to 0.05.

In the method for producing the external preparation for skin of the present invention, from the viewpoint of improving an emulsion state immediately after preparation and emulsion stability, the mass ratio (component (B2)/component (A)) of the amount of the blended component (B2) to the amount of the blended component (A) is preferably 0.005 or more, more preferably 0.01 or more, even more preferably 0.03 or more, and still even more preferably 0.05 or more, and preferably 0.30 or less, more preferably 0.20 or less, even more preferably 0.15 or less, and still even more preferably 0.10 or less. More specifically, from the viewpoint of improving an emulsion state immediately after preparation and emulsion stability, the mass ratio (component (B2)/component (A)) is preferably 0.005 to 0.30, more preferably 0.01 to 0.30, even more preferably 0.01 to 0.20, even more preferably 0.03 to 0.20, even more preferably 0.05 to 0.15, and still even more preferably 0.05 to 0.10.

Regarding the aforementioned embodiments, the present invention further discloses the following aspects.
<1> An external preparation for skin of oil-in-water type emulsification, containing components (A), (B), (C), and (D) below:
   (A): silica particles having an average particle diameter of 0.3 µm or more and 3 µm or less;
   (B): at least one selected from the group consisting of (B1) a cationic polymer having a hydrophobic moiety, and (B2) a cationic surfactant having an alkyl group;
   (C): an oily component; and
   (D): an aqueous component.
<2> The external preparation for skin of oil-in-water type emulsification according to <1>, in which the average particle diameter of the component (A) is preferably 0.4 µm or more, more preferably 0.5 µm or more, and even more preferably 0.6 µm or more, and preferably 2.9 µm or less, and more preferably 2.8 µm or less.
<3> The external preparation for skin of oil-in-water type emulsification according to <1>, in which the average particle diameter of the component (A) is preferably 0.4 µm or more and 3 µm or less, more preferably 0.5 µm or more and 3 µm or less, even more preferably 0.5 µm or more and 2.9 µm or less, and still even more preferably 0.6 µm or more and 2.8 µm or less.
<4> The external preparation for skin of oil-in-water type emulsification according to any one of <1> to <3>, in which an amount of oil absorption of the component (A) is preferably 5 mL/100 g or more, more preferably 10 mL/100 g or more, and even more preferably 20 mL/100 g or more, and preferably 700 mL/100 g or less, more preferably 600 mL/100 g or less, even more preferably 500 mL/100 g or less, even more preferably 300 mL/100 g or less, even more preferably 200 mL/100 g or less, even more preferably 170 mL/100 g or less, and still even more preferably 150 mL/100 g or less.
<5> The external preparation for skin of oil-in-water type emulsification according to any one of <1> to <4>, in which the silica particles as the component (A) are hydrophilic silica particles (i.e., surface-untreated silica particles).
<6> The external preparation for skin of oil-in-water type emulsification according to any one of <1> to <5>, in which the component (A) is aligned at an interface between the water phase and the oil phase.
<7> The external preparation for skin of oil-in-water type emulsification according to any one of <1> to <6>, in which the cationic polymer or cationic surfactant as the component (B) is adsorbed to the silica particles as the component (A).
<8> The external preparation for skin of oil-in-water type emulsification according to any one of <1> to <7>, in which the cationic group of the component (B1) is a quaternary ammonium group.
<9> The external preparation for skin of oil-in-water type emulsification according to any one of <1> to <8>, in which the component (B1) is a cationic polymer having at least one type of hydrophobic moiety selected from the group consisting of a polysiloxane moiety, a hydrocarbon moiety, and an alkyleneoxy moiety having 3 or more carbon atoms.
<10> The external preparation for skin of oil-in-water type emulsification according to any one of <1> to <9>, in which the component (B1) is at least one selected from the group consisting of a cationic silicone, a cationic vinyl polymer, and a cationized cellulose derivative.
<11> The external preparation for skin of oil-in-water type emulsification according to <10>, in which the cationic silicone is a silicone having at least one quaternary ammonium group at an end or side chain of organopolysiloxane.
<12> The external preparation for skin of oil-in-water type emulsification according to <10> or <11>, in which the cationic vinyl polymer is a vinyl polymer containing a structural unit derived from a monomer having at least one type of hydrophobic moiety selected from the group consisting of a hydrocarbon group and an alkyleneoxy group having 3 or more carbon atoms, and a structural unit derived from a monomer having a cationic group.
<13> The external preparation for skin of oil-in-water type emulsification according to any one of <10> to <12>, in which the cationized cellulose derivative is preferably a hydrophobic cationized cellulose, and more preferably at least one selected from the group consisting of a hydrophobic cationized hydroxyethyl cellulose having a long chain alkyl group, and a hydrophobic cationized hydroxyethyl cellulose having a propyleneoxy group moiety.
<14> The external preparation for skin of oil-in-water type emulsification according to any one of <1> to <13>, in which the alkyl group in the component (B2) is preferably at least one selected from the group consisting of an aliphatic alkyl group and an aromatic alkyl group, and more preferably an aliphatic alkyl group.
<15> The external preparation for skin of oil-in-water type emulsification according to any one of <1> to <14>, in which a carbon number of the alkyl group of the component (B2) is preferably 7 or more, more preferably 8 or more, even more preferably 10 or more, even more preferably 12 or more, and still even more preferably 14 or more, and preferably 22 or less.
<16> The external preparation for skin of oil-in-water type emulsification according to any one of <1> to <15>, in which the component (B2) is preferably at least one selected from the group consisting of (i) an alkyltrimethylammonium salt, (ii) an alkoxyalkyltrimethylammonium salt, (iii) a dialkyldimethylammonium salt, (iv) an alkylamidoalkyltrimethylammonium salt, (v) an alkyldimethylamine and its salt, (vi) an alkoxyalkyldimethylamine and its salt, (vii) an alkylamidoalkyldimethylamine and its salt, and (viii) a monoalkylbenzyldimethylammonium salt, more preferably at least one selected from the group consisting of (i) the alkyltrimethylammonium salt, and (iii) the dialkyldimethylammonium salt, even more preferably (iii) the dialkyldimethylammonium salt, even more preferably the dialkyldimethylammonium salt having an alkyl group having 12 or more and 22 or less carbon atoms, and still even more preferably the dialkyldimethylammonium salt having an alkyl group having 16 or more and 20 or less carbon atoms.
<17> The external preparation for skin of oil-in-water type emulsification according to any one of <1> to <16>, in which a content of the component (A) in the external preparation for skin of oil-in-water type emulsification is preferably 0.3 mass% or more, more preferably 0.5 mass% or more, even more preferably 1 mass% or more, and still even more preferably 2 mass% or more, and preferably 13 mass% or less, more preferably 10 mass% or less, even more preferably 7 mass% or less, even more preferably 5 mass% or less, and still even more preferably 4 mass% or less.
<18> The external preparation for skin of oil-in-water type emulsification according to any one of <1> to <16>, in which the content of the component (A) in the external preparation for skin of oil-in-water type emulsification is preferably 0.3 to 13 mass%, more preferably 0.5 to 10 mass%, even more preferably 1 to 10 mass%, even more preferably 1 to 7 mass%, even more preferably 1 to 5 mass%, even more preferably 2 to 5 mass%, and still even more preferably 2 to 4 mass%.
<19> The external preparation for skin of oil-in-water type emulsification according to any one of <1> to <18>, in which a content of the component (B) in the external preparation for skin of oil-in-water type emulsification is preferably 0.01 mass% or more, more preferably 0.02 mass% or more, and even more preferably 0.03 mass% or more, and preferably 1 mass% or less, more preferably 0.7 mass% or less, even more preferably 0.5 mass% or less, and still even more preferably 0.3 mass% or less.
<20> The external preparation for skin of oil-in-water type emulsification according to any one of <1> to <18>, in which the content of the component (B) in the external preparation for skin of oil-in-water type emulsification is preferably 0.01 to 1 mass%, more preferably 0.02 to 0.7 mass%, even more preferably 0.02 to 0.5 mass%, even more preferably 0.02 to 0.3 mass%, and still even more preferably 0.03 to 0.3 mass%.
<21> The external preparation for skin of oil-in-water type emulsification according to any one of <1> to <20>, in which a mass ratio (component (B)/component (A)) of the content of the component (B) to the content of the component (A) in the external preparation for skin of oil-in-water type emulsification is preferably 0.003 or more, more preferably 0.005 or more, and even more preferably 0.007 or more, and preferably 0.30 or less, more preferably 0.20 or less, even more preferably 0.10 or less, and still even more preferably 0.05 or less.
<22> The external preparation for skin of oil-in-water type emulsification according to any one of <1> to <20>, in which the mass ratio (component (B)/component (A)) of the content of the component (B) to the content of the component (A) in the external preparation for skin of oil-in-water type emulsification is preferably 0.003 to 0.30, more preferably 0.005 to 0.20, even more preferably 0.005 to 0.10, even more preferably 0.005 to 0.05, and still even more preferably 0.007 to 0.05.
<23> The external preparation for skin of oil-in-water type emulsification according to any one of <1> to <22>, in which a content of the component (B1) in the external preparation for skin of oil-in-water type emulsification is preferably 0.01 mass% or more, more preferably 0.02 mass% or more, and even more preferably 0.03 mass% or more, and preferably 1 mass% or less, more preferably 0.7 mass% or less, even more preferably 0.5 mass% or less, and still even more preferably 0.3 mass% or less.
<24> The external preparation for skin of oil-in-water type emulsification according to any one of <1> to <22>, in which the content of the component (B1) in the external preparation for skin of oil-in-water type emulsification is preferably 0.01 to 1 mass%, more preferably 0.02 to 0.7 mass%, even more preferably 0.02 to 0.5 mass%, even more preferably 0.02 to 0.3 mass%, and still even more preferably 0.03 to 0.3 mass%.
<25> The external preparation for skin of oil-in-water type emulsification according to any one of <1> to <24>, a mass ratio (component (B1)/component (A)) of the content of the component (B1) to the content of the component (A) in the external preparation for skin of oil-in-water type emulsification is preferably 0.003 or more, more preferably 0.005 or more, and even more preferably 0.007 or more, and preferably 0.30 or less, more preferably 0.20 or less, even more preferably 0.10 or less, and still even more preferably 0.05 or less.
<26> The external preparation for skin of oil-in-water type emulsification according to any one of <1> to <24>, in which the mass ratio (component (B1)/component (A)) of the content of the component (B1) to the content of the component (A) in the external preparation for skin of oil-in-water type emulsification is preferably 0.003 to 0.30, more preferably 0.005 to 0.20, even more preferably 0.005 to 0.10, even more preferably 0.005 to 0.05, and still even more preferably 0.007 to 0.05.
<27> The external preparation for skin of oil-in-water type emulsification according to any one of <1> to <26>, in which a content of the component (B2) in the external preparation for skin of oil-in-water type emulsification is preferably 0.01 mass% or more, more preferably 0.02 mass% or more, even more preferably 0.03 mass% or more, even more preferably 0.04 mass% or more, even more preferably 0.05 mass% or more, and still even more preferably 0.1 mass% or more, and preferably 1 mass% or less, more preferably 0.7 mass% or less, and even more preferably 0.5 mass% or less.
<28> The external preparation for skin of oil-in-water type emulsification according to any one of <1> to <26>, in which the content of the component (B2) in the external preparation for skin of oil-in-water type emulsification is preferably 0.01 to 1 mass%, more preferably 0.02 to 1 mass%, even more preferably 0.03 to 1 mass%, even more preferably 0.04 to 1 mass%, even more preferably 0.05 to 1 mass%, even more preferably 0.05 to 0.7 mass%, and still even more preferably 0.1 to 0.5 mass%.
<29> The external preparation for skin of oil-in-water type emulsification according to any one of <1> to <28>, in which a mass ratio (component (B2)/component (A)) of the content of the component (B2) to the content of the component (A) in the external preparation for skin of oil-in-water type emulsification is preferably 0.005 or more, more preferably 0.01 or more, even more preferably 0.03 or more, and still even more preferably 0.05 or more, and preferably 0.30 or less, more preferably 0.20 or less, even more preferably 0.15 or less, and still even more preferably 0.10 or less.
<30> The external preparation for skin of oil-in-water type emulsification according to any one of <1> to <28>, in which the mass ratio (component (B2)/component (A)) of the content of the component (B2) to the content of the component (A) in the external preparation for skin of oil-in-water type emulsification is preferably 0.005 to 0.30, more preferably 0.01 to 0.30, even more preferably 0.01 to 0.20, even more preferably 0.03 to 0.20, even more preferably 0.05 to 0.15, and still even more preferably 0.05 to 0.10.
<31> The external preparation for skin of oil-in-water type emulsification according to any one of <1> to <30>, in which the component (C) contains a liquid oil agent in an environment at 1 atmospheric pressure and 25°C.
<32> The external preparation for skin of oil-in-water type emulsification according to <31>, in which the liquid oil agent preferably contains at least one selected from the group consisting of an ester oil, a hydrocarbon oil, a silicone oil, a higher alcohol, and a higher fatty acid, more preferably contains at least one selected from the group consisting of an ester oil, a silicone oil, and a hydrocarbon oil, even more preferably contains at least one selected from the group consisting of an ester oil and a hydrocarbon oil, even more preferably contains an ester oil, and still even more preferably contains at least one selected from the group consisting of a monoester of a fatty acid having 4 or more and 18 or less carbon atoms with an alcohol having 2 or more and 24 or less carbon atoms, a diester of a fatty acid having 6 or more and 18 or less carbon atoms with a dialcohol having 2 or more and 10 or less carbon atoms, a triester of a fatty acid having 6 or more and 18 or less carbon atoms with glycerin, an ester of a fatty acid having 6 or more and 18 or less carbon atoms with pentaerythritol, a diester of a dicarboxylic acid having 2 or more and 18 or less carbon atoms with a branched alcohol having 2 or more and 18 or less carbon atoms, and an (C12-C15) alkyl benzoate.
<33> The external preparation for skin of oil-in-water type emulsification according to <31> or <32>, in which a content of the liquid oil agent in the external preparation for skin of oil-in-water type emulsification is preferably 1 mass% or more, more preferably 2 mass% or more, and even more preferably 3 mass% or more, and preferably 30 mass% or less, more preferably 25 mass% or less, even more preferably 20 mass% or less, and still even more preferably 10 mass% or less.
<34> The external preparation for skin of oil-in-water type emulsification according to <31> or <32>, in which the content of the liquid oil agent in the external preparation for skin of oil-in-water type emulsification is preferably 1 to 30 mass%, more preferably 1 to 25 mass%, even more preferably 2 to 25 mass%, even more preferably 2 to 20 mass%, and still even more preferably 3 to 10 mass%.
<35> The external preparation for skin of oil-in-water type emulsification according to any one of <31> to <34>, in which when the liquid oil agent contains an ester oil and a hydrocarbon oil, a total content of the ester oil and the hydrocarbon oil in the liquid oil agent is preferably 60 mass% or more, more preferably 70 mass% or more, even more preferably 80 mass% or more, even more preferably 90 mass% or more, and still even more preferably 95 mass% or more.
<36> The external preparation for skin of oil-in-water type emulsification according to any one of <31> to <34>, in which when the liquid oil agent contains an ester oil and a hydrocarbon oil, the total content of the ester oil and the hydrocarbon oil in the liquid oil agent is preferably 60 to 100 mass% or more, more preferably 70 to 100 mass% or more, even more preferably 80 to 100 mass% or more, even more preferably 90 to 100 mass% or more, and still even more preferably 95 to 100 mass%.
<37> The external preparation for skin of oil-in-water type emulsification according to any one of <31> to <36>, in which when the liquid oil agent contains an ester oil, the content of the ester oil in the liquid oil agent is preferably 40 mass% or more, more preferably 50 mass% or more, even more preferably 60 mass% or more, even more preferably 70 mass% or more, even more preferably 80 mass% or more, even more preferably 90 mass% or more, and still even more preferably 95 mass% or more.
<38> The external preparation for skin of oil-in-water type emulsification according to any one of <31> to <36>, in which when the liquid oil agent contains an ester oil, the content of the ester oil in the liquid oil agent is preferably 40 to 100 mass% or more, more preferably 50 to 100 mass% or more, even more preferably 60 to 100 mass% or more, even more preferably 70 to 100 mass% or more, even more preferably 80 to 100 mass%, even more preferably 90 to 100 mass%, and still even more preferably 95 to 100 mass%.
<39> The external preparation for skin of oil-in-water type emulsification according to any one of <1> to <38>, in which the component (C) contains a solid oil agent in an environment at 1 atmospheric pressure and 25°C.
<40> The external preparation for skin of oil-in-water type emulsification according to <39>, in which a content of the solid oil agent in the external preparation for skin of oil-in-water type emulsification is preferably 0.1 mass% or more, more preferably 0.2 mass% or more, and even more preferably 0.3 mass% or more, and preferably 3 mass% or less, more preferably 2 mass% or less, and even more preferably 1 mass% or less.
<41> The external preparation for skin of oil-in-water type emulsification according to any one of <1> to <40>, in which a content of the component (C) in the external preparation for skin of oil-in-water type emulsification is preferably 5 mass% or more, more preferably 10 mass% or more, and even more preferably 15 mass% or more, and preferably 40 mass% or less, more preferably 30 mass% or less, even more preferably 25 mass% or less, and still even more preferably 20 mass% or less.
<42> The external preparation for skin of oil-in-water type emulsification according to any one of <1> to <40>, in which the content of the component (C) in the external preparation for skin of oil-in-water type emulsification is preferably 5 to 40 mass%, more preferably 10 to 30 mass%, even more preferably 10 to 25 mass%, and still even more preferably 15 to 20 mass%.
<43> The external preparation for skin of oil-in-water type emulsification according to any one of <1> to <42>, in which the component (C) contains at least one selected from the group consisting of an ultraviolet absorber and an ester oil.
<44> The external preparation for skin of oil-in-water type emulsification according to <43>, in which a total content of the ultraviolet absorber and the ester oil in the external preparation for skin of oil-in-water type emulsification is preferably 5 mass% or more, more preferably 10 mass% or more, and even more preferably 15 mass% or more, and preferably 30 mass% or less, more preferably 25 mass% or less, and even more preferably 20 mass% or less.
<45> The external preparation for skin of oil-in-water type emulsification according to <43>, in which the total content of the ultraviolet absorber and the ester oil in the external preparation for skin of oil-in-water type emulsification is preferably 5 to 30 mass%, more preferably 10 to 25 mass%, and even more preferably 15 to 20 mass%.
<46> The external preparation for skin of oil-in-water type emulsification according to any one of <1> to <45>, in which the component (D) contains at least one of water and the water-soluble organic solvent.
<47> The external preparation for skin of oil-in-water type emulsification according to <46>, in which the water-soluble organic solvent is preferably at least one selected from the group consisting of a saturated monohydric alcohol having 1 or more and 3 or less carbon atoms and a polyhydric alcohol, more preferably at least one selected from the group consisting of ethanol, isopropyl alcohol, ethylene glycol, diethylene glycol, propylene glycol, dipropylene glycol, 1,3-butylene glycol, and glycerin, and even more preferably at least one selected from the group consisting of ethanol, dipropylene glycol, 1,3-butylene glycol, and glycerin.
<48> The external preparation for skin of oil-in-water type emulsification according to any one of <1> to <47>, in which a content of the component (D) in the external preparation for skin of oil-in-water type emulsification is preferably 53 mass% or more, more preferably 55 mass% or more, and even more preferably 57 mass% or more, and preferably 80 mass% or less, more preferably 75 mass% or less, and even more preferably 73 mass% or less.
<49> The external preparation for skin of oil-in-water type emulsification according to any one of <1> to <47>, in which a content of the component (D) in the external preparation for skin of oil-in-water type emulsification is preferably 53 to 80 mass%, more preferably 55 to 75 mass%, and even more preferably 57 to 73 mass%.
<50> The external preparation for skin of oil-in-water type emulsification according to any one of <1> to <49>, further containing a component (E) as a thickener.
<51> The external preparation for skin of oil-in-water type emulsification according to <50>, in which the component (E) preferably contains one or more selected from the group consisting of the natural macromolecule, the semisynthetic macromolecule, and the synthetic macromolecule, more preferably contains at least one selected from the group consisting of xanthan gum, hydroxyethyl cellulose, and the vinyl polymer containing a structural unit derived from a (meth)acrylic acid or a structural unit derived from a derivative of a (meth)acrylic acid, even more preferably contains xanthan gum, and the vinyl polymer containing a structural unit derived from a (meth)acrylic acid or a structural unit derived from a derivative of a (meth)acrylic acid, even more preferably contains at least one selected from the group consisting of xanthan gum, and the vinyl polymer containing a structural unit derived from at least one type of acrylamide-based monomer selected from the group consisting of acrylamide and AMPS and their salt, and still even more preferably contains at least one selected from the group consisting of xanthan gum, polyacrylamide, polyacryloyl dimethyl tauric acid, and the copolymer containing a structural unit derived from at least one type of acrylamide-based monomer selected from the group consisting of acrylamide and AMPS, and their salt.
<52> The external preparation for skin of oil-in-water type emulsification according to <50> or <51>, in which a content of the component (E) in the external preparation for skin of oil-in-water type emulsification is preferably 0.05 mass% or more, more preferably 0.10 mass% or more, and even more preferably 0.13 mass% or more, and preferably 3 mass% or less, more preferably 2 mass% or less, and even more preferably 1.5 mass% or less.
<53> The external preparation for skin of oil-in-water type emulsification according to <50> or <51>, in which the content of the component (E) in the external preparation for skin of oil-in-water type emulsification is preferably 0.05 to 3 mass%, more preferably 0.10 to 2 mass%, and even more preferably 0.13 to 1.5 mass%.
<54> A method for producing the external preparation for skin of oil-in-water type emulsification according to any one of <1> to <53>, the method including a step 1 of mixing the component (A) with the component (B) in water.
<55> The method for producing the external preparation for skin of oil-in-water type emulsification according to <54>, further including, after a mixture in which the components (A) and (B) are mixed in water in the step 1 is obtained, a step of mixing and emulsifying the mixture, and a water phase component and an oil phase component.
<56> The method for producing the external preparation for skin of oil-in-water type emulsification according to any one of <1> to <55>, including steps I to IV
   Step I: a step of adding the component (A) to the component (D), then adding the component (B) diluted with the component (D), mixing the components, to obtain a mixture of the components (A) and (B)
   Step II: mixing the component (D) and if necessary, the thicker as the component (E) and the optional water-soluble component, to obtain a water phase component
   Step III: mixing the component (C) and if necessary, the aforementioned optional component, to obtain an oil phase component
   Step IV: adding the mixture of the components (A) and (B) obtained in the step I and the oil phase component obtained in the step III to the water phase component obtained in the step II, followed by mixing and emulsifying, to obtain the external preparation for skin
<57> The method for producing the external preparation for skin of oil-in-water type emulsification according to any one of <1> to <55>, including steps I' to IV'.
   Step I': a step of adding the component (A) to the component (D), then adding the component (B) diluted with the component (D), mixing the components, to obtain a mixture of the components (A) and (B)
   Step II': mixing the component (D) and if necessary, the thickener as the component (E) and the optional water-soluble component in the mixture obtained in the step I', to obtain a water phase component
   Step III': mixing the component (C) and if necessary, the optional oil-soluble component, to obtain an oil phase component
   Step IV': adding the oil phase component obtained in the step III' to the water phase component obtained in the step II', followed by mixing and emulsifying, to obtain the external preparation for skin

### Examples

In the following Examples and Comparative Examples, "part" and %" are "part by mass" and "mass%", respectively, unless otherwise indicated.

### (Average Particle Diameter of Component (A))

0.1 g of the component (A) was added to 10 g of purified water, the mixture is subjected to ultrasonic dispersion for 1 minute, and a volume average particle diameter (D₅₀) was measured with a laser diffraction/scattering particle diameter dispersion measurement device "LA-960" (manufactured by Horiba, Ltd.) as an average particle diameter.

Provided that when the volume average particle diameter (D₅₀) is less than 0.1 µm, under conditions with transmission electron microscope (TEM) at an observation magnification of about 50,000 to 100,000, the number-average value of primary particle diameters of about 300 primary particles in the observation image was calculated as the average particle diameter.

### (Number-average Molecular Weight of Poly(N-acylalkyleneimine))

The number-average molecular weight was determined by gel permeation chromatography (GPC) under the following measurement conditions.

### [Measurement Conditions]

Column: two columns GPC K-804L (product name, manufactured by Showa Denko K.K.) are connected in series
Eluent: 1 mmol/L FARMIN DM20 (product name, available from Kao Corporation)/chloroform
Flow rate: 1.0 mL/min
Column temperature: 40°C
Detector: differential refractive index detector (RID)
Sample concentration: 5 mg/mL
Sample injection amount: 100 µL
Standard substance: polystyrene

### Production Example 1

In 606 g of dehydrated ethyl acetate, 200 g (1.98 mol) of 2-ethyl-2-oxazoline and 12 g (0.077 mol) of diethyl sulfate were dissolved, and heated and refluxed in a nitrogen atmosphere for 8 hours, to obtain poly(N-propionylethyleneimine) (number-average molecular weight: 2,500). To the solution, 200 g of side chain primary aminopropyl-modified dimethyl polysiloxane (weight-average molecular weight: 26,000, amine equivalent weight: 2,000) dissolved in 606 g of dehydrated ethyl acetate was added, and heated and refluxed in a nitrogen atmosphere for 12 hours, and the solvent was removed by an evaporator, to obtain poly(N-propionylethyleneimine)/dimethyl polysiloxane copolymer B1-1 as a pale yellow rubbery semisolid. The mass ratio (content of segment (a)/total content of segments (a) and (b)) in the obtained poly(N-propionylethyleneimine)/dimethyl polysiloxane copolymer B1-1 was 0.50, and the weight-average molecular weight (Mwt) was 60,000 (calculated value).

### Production Example 2

In 67.7 g of dehydrated ethyl acetate, 27.2 g (0.27 mol) of 2-ethyl-2-oxazoline and 6.2 g (0.040 mol) of diethyl sulfate were dissolved, and heated and refluxed in a nitrogen atmosphere for 8 hours, to obtain poly(N-propionylethyleneimine) (number-average molecular weight: 800). To the solution, 100 g of side chain primary aminopropyl-modified dimethyl polysiloxane (weight-average molecular weight: 50,000, amine equivalent weight: 2,000) was added, to obtain poly(N-propionylethyleneimine)/dimethyl polysiloxane copolymer B 1-2 as a pale yellow rubbery semisolid. The mass ratio (content of segment (a)/total content of segments (a) and (b)) in the obtained poly(N-propionylethyleneimine)/dimethyl polysiloxane copolymer B1-2 was 0.75, and the weight-average molecular weight (Mwt) was 70,000 (calculated value).

### Examples 1 to 20 and Comparative Examples 1 to 15

Each external preparation for skin was obtained so as to have each blending composition shown in Tables 1 to 6 by the following method.

To purified water, the component (A) was added with stirring at a predetermined temperature of about 25°C. Subsequently, the component (B) diluted with purified water or ethanol in advance was added with stirring, the component (A) and the component (B) were mixed, and the component (B) was adsorbed to the surface of the component (A), to obtain a mixture of the components (A) and (B) (Step I).

To purified water, the component (E) and an additional aqueous component were added with stirring at a predetermined temperature of about 25°C, to prepare a water phase component (Step II).

The component (C) was uniformly dissolved at a predetermined temperature of about 80°C, and if necessary, hydrophobized metal oxide fine particles were added as an ultraviolet scattering agent with stirring, to prepare an oil phase component (Step III).

The mixture of the components (A) and (B) obtained in the step I and the oil phase component obtained in the step III were added at a predetermined temperature of about 25°C to the water phase component obtained in the step II, followed by mixing and emulsifying, to obtain each external preparation for skin (Step IV).

The blending compositions (mass%) of components shown in Tables 1 to 6 are active amounts.

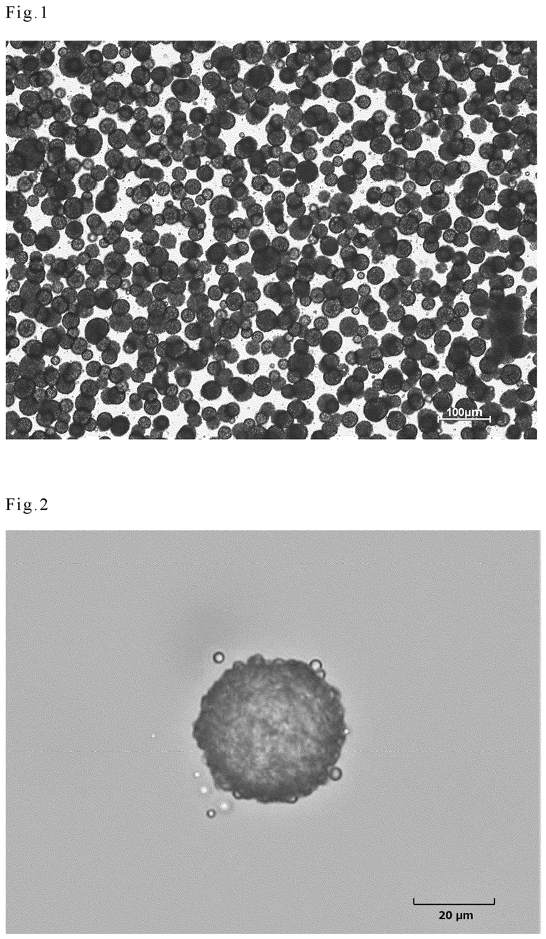

FIG. 1 shows a photomicrograph obtained by photographing the emulsion state of the external preparation for skin obtained in Example 1, and FIG. 2 shows a photomicrograph obtained by photographing the emulsion state of a dilution obtained by diluting the external preparation for skin obtained in Example 1 50 times with water.

### In Tables 1 to 6, each notation is as follows.

### (Component (A))

GODDBALL E-2C: available from SUZUKIYUSHI INDUSTRIAL CORPORATION, surface-untreated product, oil absorption amount: 150 mL/100 g (published value), specific surface area: 400 mm²/g (published value)
SILICA MICRO BEAD P-500: available from JGC Catalysts and Chemicals Ltd., surface-untreated product, oil absorption amount: 60 mL/100 g (published value), specific surface area: 150 mm²/g (published value)
COSMO55: available from JGC Catalysts and Chemicals Ltd., surface-untreated product, oil absorption amount: 30 mL/100 g (published value)
GODDBALL G-2C: available from SUZUKIYUSHI INDUSTRIAL CORPORATION, surface-untreated product, oil absorption amount: 20 mL/100 g (published value), specific surface area: 5 mm²/g (published value)

### (Component (A'))

FINEX-33W: available from Sakai Chemical Industry Co., Ltd.
TIPAQUE PFC407: available from ISHIHARA SANGYO KAISHA, LTD.
AEROSIL 200: available from NIPPON AEROSIL CO., LTD.
SUNSPHARE NP-30: available from AGC Si-Tech Co., Ltd.
MP-100: available from Tayca Corporation

### (Component (B 1))

ABIL Quat 3272: available from Evonik Industries AG.
ABIL T Quat 60: available from Evonik Industries AG.
ABIL UV Quat 50: available from Evonik Industries AG.
PLASCIZE L-514: available from GOO Chemical Co., Ltd.
PLASCIZE L-450W: available from GOO Chemical Co., Ltd.
PPG-2 hydroxypropyl trimonium cellulose: cationized ethyleneoxy group substitution degree: 0.2, substitution degree of propyleneoxy group: 2.2, average degree of polymerization: 800, Z⁻ represented in general formula (4-6): chloride ion

### (Component (B2))

VARISOFT TA 100: available from Evonik Industries AG.

### (Component (B'))

PLASCIZE L-440W: available from GOO Chemical Co., Ltd.
MERQUAT 100: available from Lubrizol Corporation
MERQUAT 550: available from Lubrizol Corporation
MERQUAT 295: available from Lubrizol Corporation
MERQUAT PLUS 3331: available from Lubrizol Corporation
MERQUAT 2001: available from Lubrizol Corporation
GAFQUAT 755N: available from Ashland Inc.

### (Component (C))

UVINUL MC80: available from BASF
UVINUL A PLUS GRANULAR: available from BASF
UVINUL T-150: available from BASF
TINOSORB S: : available from BASF
EXCEPEAL IPP: available from Kao Corporation
PARLEAM 4: available from NOF CORPORATION
SILICONE KF-96A-6CS: available from Shin-Etsu Chemical Co., Ltd.
SALACOS HS-6C: available from The Nisshin OilliO Group, Ltd.
SALACOS WO-6: available from The Nisshin OilliO Group, Ltd.

### (Component (D))

1,3-BUTYLENE GLYCOL-P: available from KH Neochem Co., Ltd.
ETHANOL (95%): available from Japan Synthetic Alcohol Co., Ltd.

### (Component (E))

SIMULGEL EG: available from SEPPIC
SEPINOV EMT 10: available from SEPPIC
KELDENT: available from MP Gokyo Food & Chemical Co., Ltd.

### (Other component)

FINEX-50-OTS: available from Sakai Chemical Industry Co., Ltd.
FINEX-50-LPTM: available from Sakai Chemical Industry Co., Ltd.
CLEWAT N: available from Nagase & Co., Ltd.

(1) An emulsion state and (2) emulsion stability shown below were evaluated using the obtained external preparations for skin. The results are shown in Tables 1 to 6.

### <Evaluation>

### (1) Emulsion state

An emulsion state when 2 hours passed after the external preparation for skin in each of Examples and Comparative Examples was prepared was observed with an optical microscope, and evaluated in accordance with the following criteria.

### [Evaluation criteria]

4: emulsion particles were uniform, and coalescence or aggregation was not recognized.
3: emulsion particles were generally uniform, but coalescence and aggregation were slightly recognized.
2: emulsion particles were not uniform, and coalescence and aggregation were recognized.
1: emulsification was not achieved, and separation of an oil phase was immediately recognized. Alternatively, a water-in-oil type emulsion is obtained.

### (2) Emulsion stability

50 g of the external preparation for skin in each of Examples and Comparative Examples was put into a glass beaker, and stirred by low propeller rotation at 10 rpm for 1 hour. Subsequently, an emulsion state was evaluated in accordance with the following criteria by visual inspection.

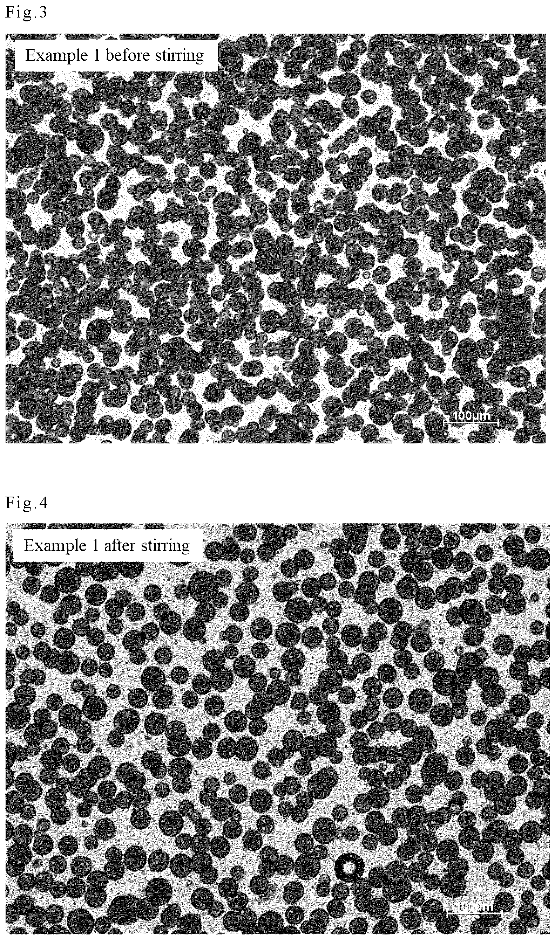

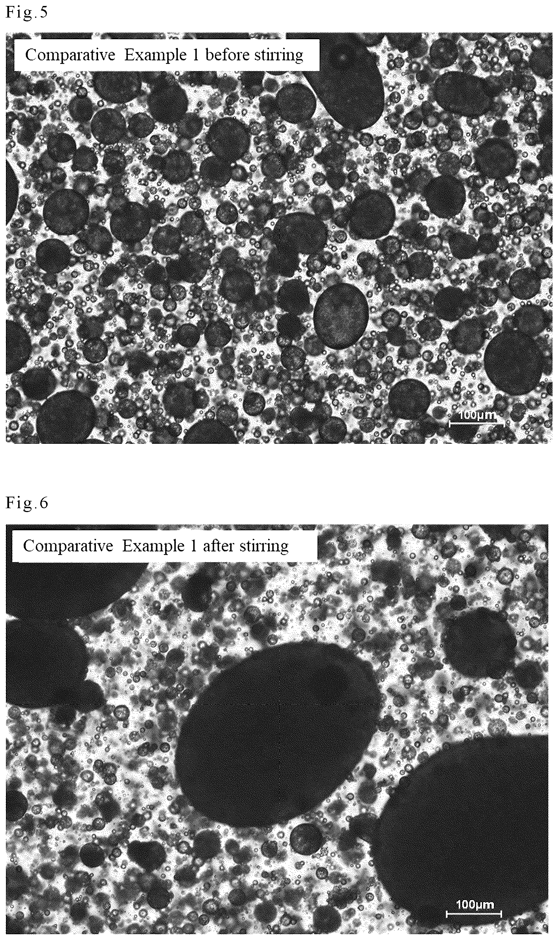

FIGs. 3 to 6 are each a photomicrograph illustrating the emulsion state before and after stirring in evaluation of emulsion stability in Example 1 and Comparative Example 1.

### [Evaluation criteria]

4: a change in the emulsion state was not recognized.
3: coalescence of emulsion particles was slightly recognized.
2: coalescence of emulsion particles was recognized.
1: separation of an oil phase or an oil stain was recognized.

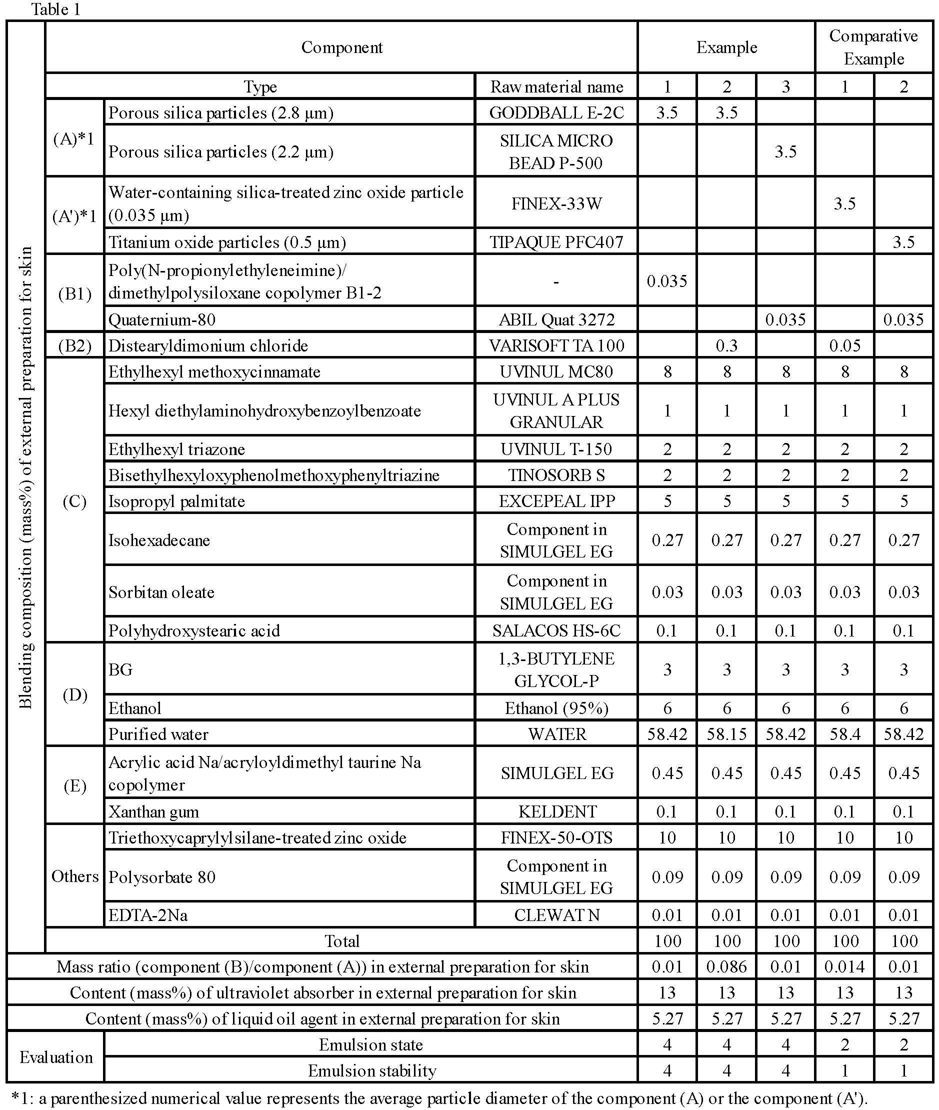

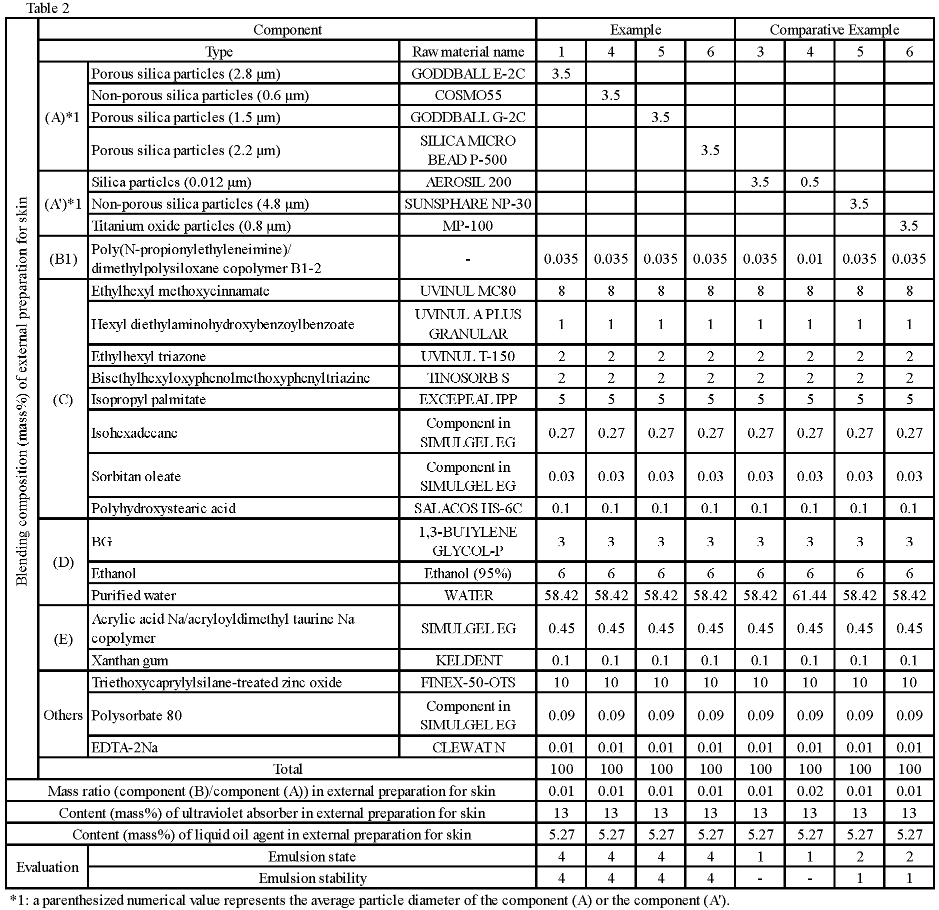

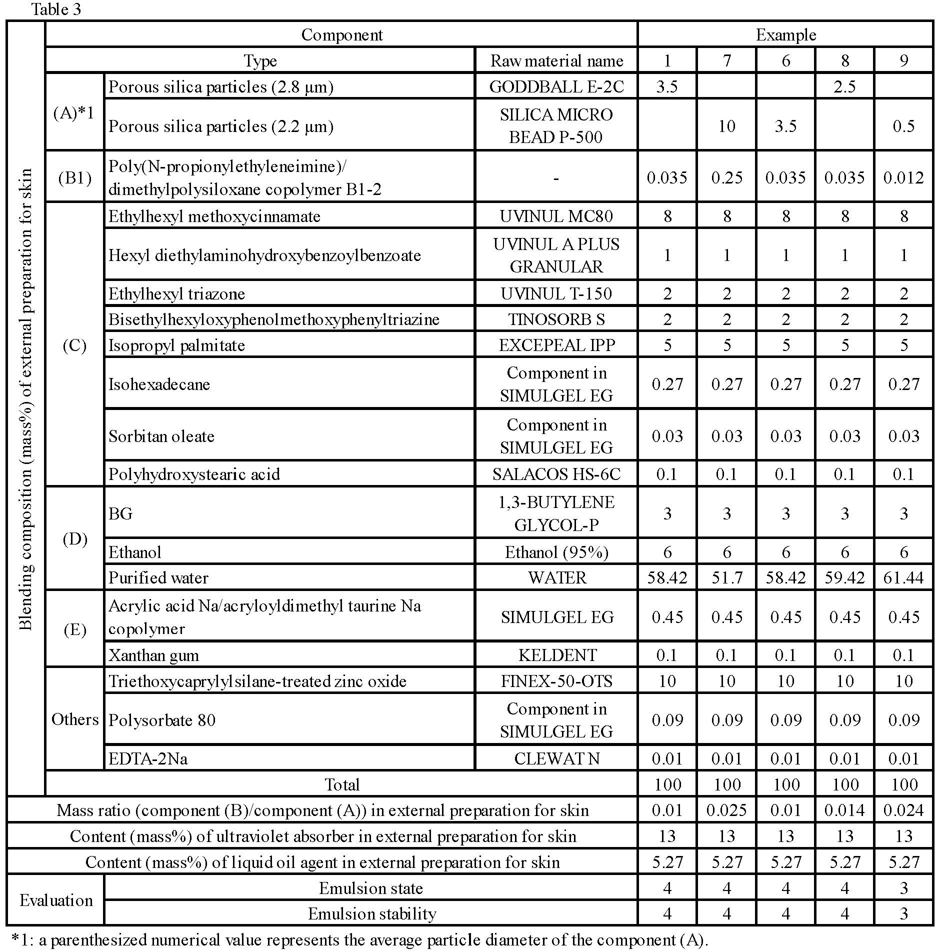

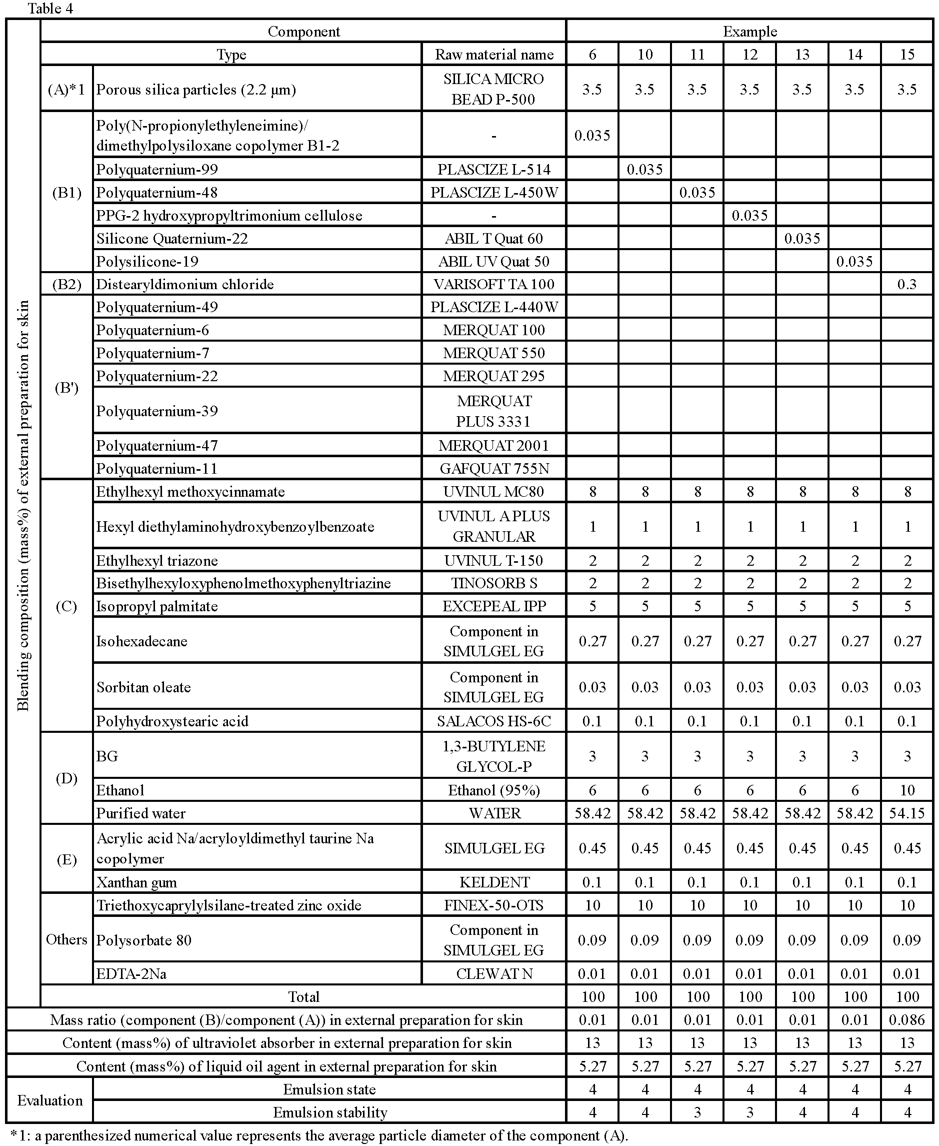

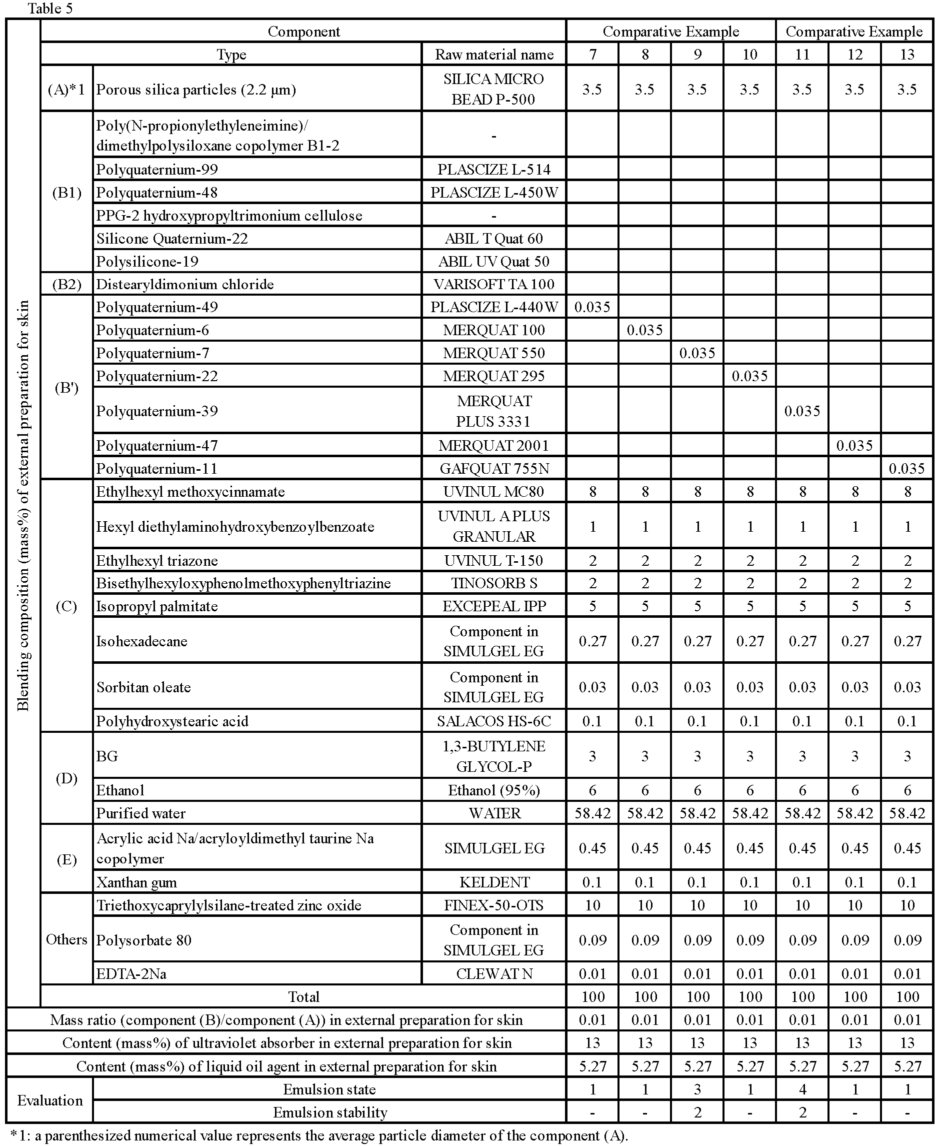

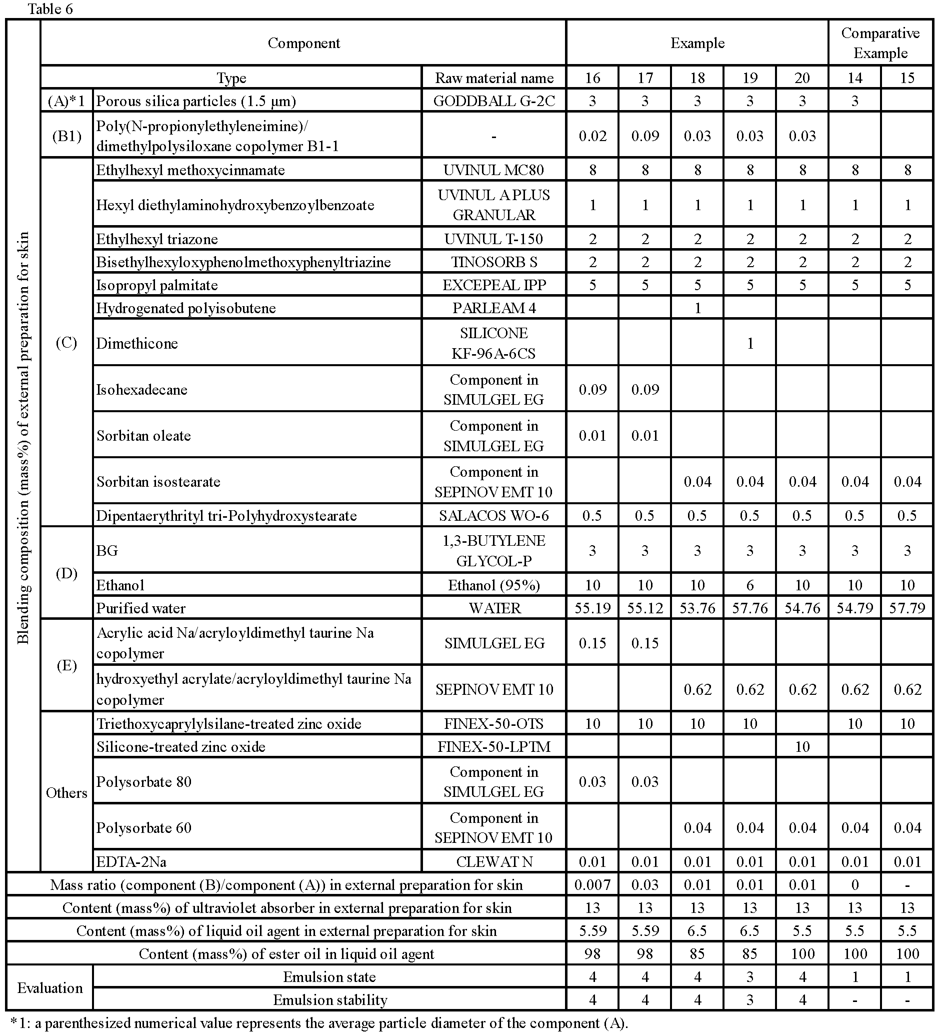

As seen from Tables 1 to 6, the emulsion state and the emulsion stability in Examples 1 to 20 are more excellent than those in Comparative Examples 1 to 15 since the components (A) to (D) are contained, the average particle diameter of the silica particles as the component (A) is 0.3 µm or more and 3 µm or less, and the component (B) is a specific cationic compound.

As seen from FIG. 1, the external preparation for skin in Example 1 has an emulsion form in which a dispersion phase including oil droplets is formed in a water phase that is a continuous phase. As confirmed from FIG. 2, the external preparation for skin in Example 1 has a so-called Pickering emulsion form in which the silica particles as the component (A) are aligned at the interface between the water phase and the oil phase.

In this specification, as one embodiment according to the present invention, Tables 7 and 8 below disclose Prescription Examples 1 to 13.

In Tables 7 and 8, each notation other than those listed in Tables 1 to 6 is as follows.

### (Component (B2))

Microcare Quat EQG: available from THOR japan
VARISOFT 432 PPG: available from Evonik Industries AG.
VARISOFT BT 85 Pellets: available from Evonik Industries AG.
VARISOFT 300: available from Evonik Industries AG.
EMPIGEN BAC 50: available from HUNTSMAN Corporation (Component (C))
PARSOL 1789: available from DSM
PARSOL HMS: available from DSM
PARSOL EHS: available from DSM
PARSOL 340: available from DSM
CERAPHYL SLK: available from Ashland Inc.
DOWSIL 556 COSMETIC GRADE FLUID: available from Dow Chemical Company
BEHENYL ALCOHOL 65: available from Nikko Chemicals Co., Ltd.
DSP-OL 100: available from INNOSPEC ACTIVE CHEMICALS
ESTEMOL N-01: available from The Nisshin OilliO Group, Ltd.
RHEOPEARL KL2: available from Chiba Flour Milling Co., Ltd.
RHEODOL SP-S10V: available from Kao Corporation

### (Component (D))

DPG-RF: available from ADEKA Corporation

### (Component (E))

VOLAREST FL: available from Croda Japan
SEPILIFE NUDE: available from SEPPIC
PEMULEN TR-1: available from The Lubrizol Corporation

### (Other component)

ZANO 10 PLUS: available from EverCare
Micro TiO₂ 035 AS: available from K.S.PEARL
GCG50TRSG: available from Kobo Products, Inc.
GCG50TRYSG: available from Kobo Products, Inc.
RonaFlair Balance Green: available from Merck KGaA
SUNSIL 130NP: available from Sunjin Beauty Science
AMP-ULTRA PC 1000: available from ANGUS CHEMICAL COMPANY
STR-100W-OTS: available from Sakai Chemical Industry Co., Ltd.
MT-N1: available from Tayca Corporation
KOH (48%): available from AGC Inc.

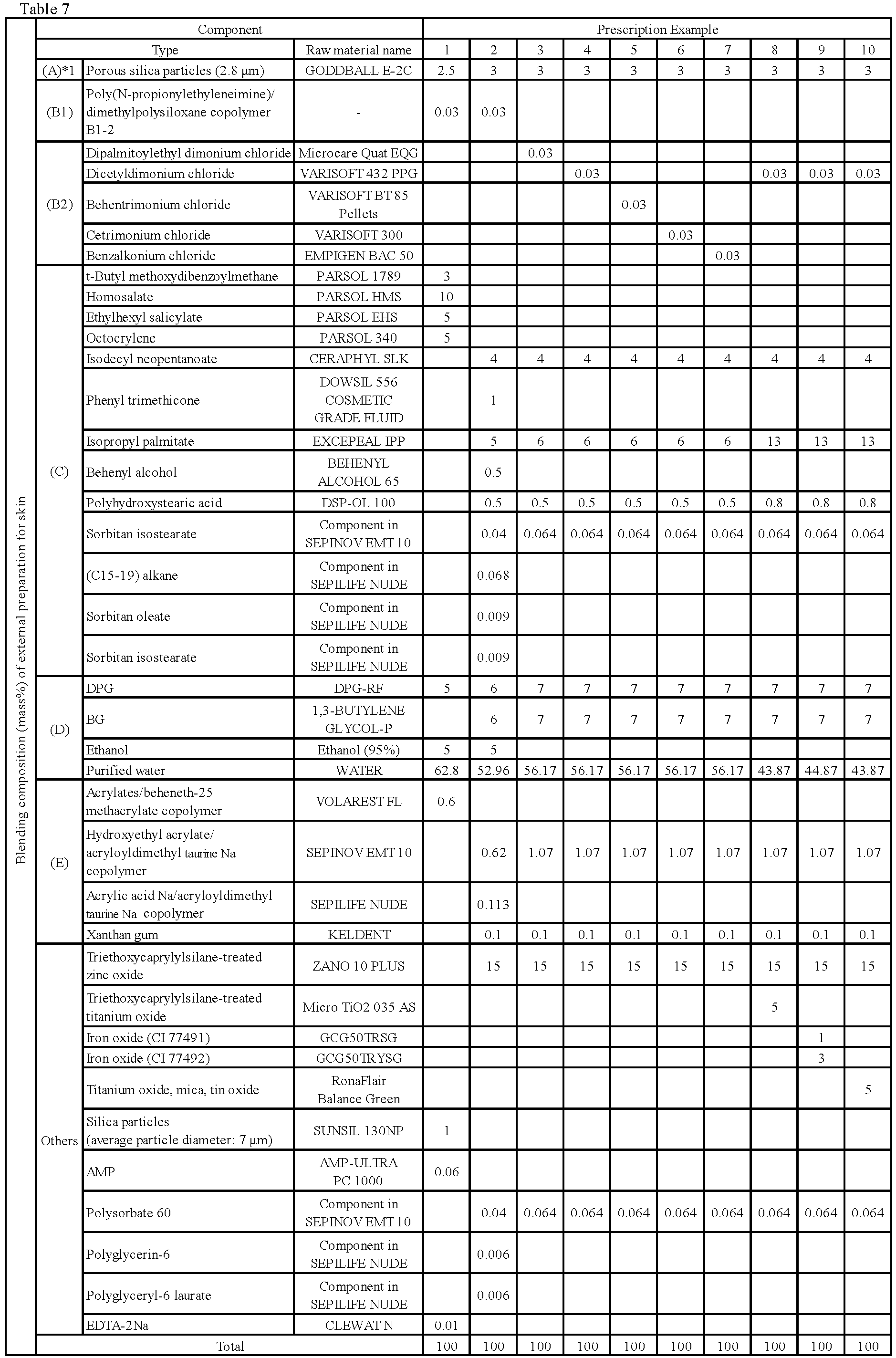

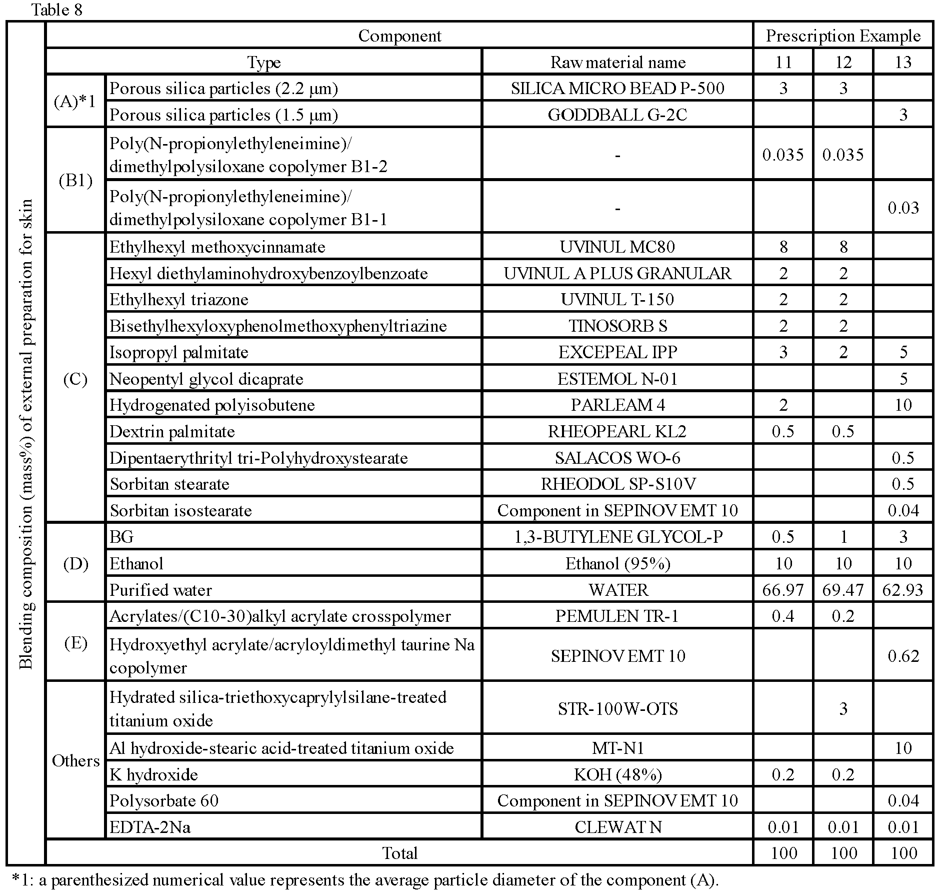

### Industrial Applicability

The present invention can provide the external preparation for skin of oil-in-water type emulsification that is excellent in an emulsion state immediately after preparation and emulsion stability. The external preparation for skin of oil-in-water type emulsification is especially useful as a skin cosmetic.

## Claims

1. An external preparation for skin of oil-in-water type emulsification, comprising components (A), (B), (C), and (D) below:
(A): silica particles having an average particle diameter of 0.3 µm or more and 3 µm or less;
(B): at least one selected from the group consisting of (B1) a cationic polymer having a hydrophobic moiety, and (B2) a cationic surfactant having an alkyl group;
(C): an oily component; and
(D): an aqueous component.

2. The external preparation for skin of oil-in-water type emulsification according to claim 1, wherein the component (A) is aligned at an interface between the water phase and the oil phase.

3. The external preparation for skin of oil-in-water type emulsification according to claim 1 or 2, wherein the cationic polymer or the cationic surfactant as the component (B) is adsorbed to the silica particles as the component (A).

4. The external preparation for skin of oil-in-water type emulsification according to any one of claims 1 to 3, further comprising a component (E) as a thickener.

5. The external preparation for skin of oil-in-water type emulsification according to any one of claims 1 to 4, wherein the component (B1) is a cationic polymer having at least one type of hydrophobic moiety selected from the group consisting of a polysiloxane moiety, a hydrocarbon moiety, and an alkyleneoxy moiety having 3 or more carbon atoms.

6. The external preparation for skin of oil-in-water type emulsification according to any one of claims 1 to 5, wherein the component (B 1) is at least one selected from the group consisting of a cationic silicone, a cationic vinyl polymer, and a cationized cellulose derivative.

7. The external preparation for skin of oil-in-water type emulsification according to any one of claims 1 to 6, wherein a content of the component (A) is 0.5 mass% or more.

8. The external preparation for skin of oil-in-water type emulsification according to any one of claims 1 to 7, wherein the component (C) contains at least one selected from the group consisting of an ultraviolet absorber and an ester oil.

9. A method for producing the external preparation for skin of oil-in-water type emulsification according to any one of claims 1 to 8, the method comprising a step 1 of mixing the component (A) with the component (B) in water.
